# EUROPEAN PATENT SPECIFICATION

(11) **EP 3 164 046 B1**
(45) Date of publication and mention of the grant of the patent: **28.10.2020**
(21) Application number: 15814823.9
(22) Date of filing: 02.07.2015
(51) Int. Cl.: G01N 21/65, G01J 3/02, A61B 1/018, A61B 5/00, G01J 3/44, G01N 21/27

(54) **RAMAN SPECTROSCOPY SYSTEM, APPARATUS, AND METHOD FOR ANALYZING, CHARACTERIZING, AND/OR DIAGNOSING A TYPE OR NATURE OF A SAMPLE OR A TISSUE SUCH AS AN ABNORMAL GROWTH**
RAMANSPEKTROSKOPIESYSTEM, VORRICHTUNG UND VERFAHREN ZUR ANALYSE, CHARAKTERISIERUNG UND/ODER DIAGNOSE EINES TYPS ODER EINER ART EINER PROBE ODER EINES GEWEBES, WIE ETWA ABNORMALES WACHSTUM
SYSTÈME DE SPECTROSCOPIE RAMAN, APPAREIL ET PROCÉDÉ D'ANALYSE, CARACTÉRISATION ET/OU DIAGNOSTIC D'UN TYPE OU D'UNE NATURE D'UN ÉCHANTILLON OU D'UN TISSU TEL QU'UNE CROISSANCE ANORMALE

(30) Priority: 02.07.2014 GB 201411800; 02.07.2014 GB 201411803; 02.07.2014 GB 201411820
(43) Date of publication of application: 10.05.2017
(73) Proprietor: National University of Singapore, Singapore 119077 (SG)
(72) Inventor: HUANG, Zhiwei, Singapore 119077 (SG)
(74) Representative: Puschmann Borchert Kaiser Klettner Patentanwälte Partnerschaft mbB
(86) International application number: PCT/SG2015/050195
(87) International publication number: WO 2016/003371

(56) References cited:
- WO-A1-2014/007759
- WO-A1-2014/007759
- WO-A1-2014/129970
- JP-A- 2007 090 044
- JP-A- 2007 090 044
- US-A1- 2010 262 378
- US-A1- 2013 231 573
- SHIYAMALA DURAIPANDIAN ET AL: "Simultaneous Fingerprint and High-Wavenumber Confocal Raman Spectroscopy Enhances Early Detection of Cervical Precancer In Vivo", ANALYTICAL CHEMISTRY, vol. 84, no. 14, 17 July 2012 (2012-07-17) , pages 5913-5919, XP055250867, ISSN: 0003-2700, DOI: 10.1021/ac300394f
- DURAIPANDIAN,S ET AL.: 'Simultaneous Fingerprint and High-Wavenumber Confocal Raman Spectroscopy Enhances Early Detection of Cervical Precancer In Vivo' ANALYTICAL CHEMISTRY vol. 84, no. 14, 20 July 2012, pages 5913 - 5919, XP055250867
- BERGHOLT,MS ET AL.: 'Development of a Multiplexing Fingerprint and High Wavenumber Raman Spectroscopy Technique for Real-Time In Vivo Tissue Raman Measurements at Endoscopy' JOURNAL OF BIOMEDICAL OPTICS vol. 18, no. 3, 28 February 2013, page 030502, XP060024068

## Description

### Background art

Aspects of the present disclosure relate to a Raman spectroscopy system and method for enhanced accuracy identification of a type or nature of a sample or a tissue to which excitation energy (e.g., collimated illumination) is directed, such as an abnormal or apparently abnormal growth (e.g., as cancer). In particular, but not exclusively, aspects of the present disclosure enable real-time enhanced accuracy diagnosis of abnormal tissues such as gastrointestinal growths, *in vivo* and *ex vivo.*

It is highly desirable to accurately and rapidly characterize and identify the nature of tissues such as neoplastic gastrointestinal growths by way of minimally invasive techniques such as endoscopy. For instance, colorectal cancer (CRC) is a common disease with a high mortality rate when discovered in the late stage. The identification and eradication of neoplastic polyps is one of the most important measures to reduce colorectal mortality and morbidity. Differentiation between hyperplastic polyps that pose little or no risk of malignant transformation and adenomas with prominent malignant latency remains a clinical challenge using conventional colonoscopy techniques.

With over 1.2 million new cancer cases and 608,700 deaths estimated to occur annually, CRC is a major problem in the modern world. Early identification of precancerous polyps (i.e., adenoma) in the curable stages together with appropriate therapeutic interventions, such as polypectomies or endoscopic mucosal resections (EMRs), remain the most important measures to reduce colorectal mortality and morbidity. The existing colonoscopic approaches suffer from a number of fundamental clinical limitations. This is because conventional colonoscopy entirely relies on the visualization of gross mucosal features of polyps, such as pit patterns, vascular patterns, etc. and provides little or no bio-molecular information about the tissue. Current standards of care therefore recommend resection of all suspicious polypoid lesions or abnormal growths identified during colorectal examinations. This approach is labor intensive; results in high cost histopathological assessments; and results in unnecessary risk to the patient since one-third to one-half of all polypoid lesions turn out to be hyperplastic. Although the absolute risk of polypectomy is considered relatively small, it still remains the most common cause of complication, such as bleeding, perforation, etc. during colonoscopy. Taking into account the existing clinical challenges and recent introduction of widespread colorectal population-based screening programs, the need for advanced endoscopic approaches has never been greater. Recent research has thus been directed towards development of more sophisticated molecular imaging and spectroscopy techniques for improving *in vivo* diagnosis and analysis.

Evidence has confirmed that the application of *ex vivo* Raman spectroscopy on colon tissue specimens provides encouraging accuracies, ranging from 89% to 99% for discrimination between different pathological types (i.e., normal, hyperplastic polyps, adenoma and adenocarcinoma). This still requires the removal of the polyps in order for the Raman spectroscopy to identify the nature of the pathological types and thus has the same problems as the conventional endoscopic techniques mentioned above. In addition, there have been many barriers to the translation of Raman spectroscopy into *in vivo* clinical diagnostics. These include technical limitations such as inherently weak tissue Raman scattering, lengthy acquisition times (>5 s) and a fundamental necessity for developing long (>1.9 m) miniaturized fiber-optic probe designs with low fused-silica interference, high collection efficiency and depth resolving capability. The probe needs to be made with low fused-silica as fused-silica has a strong Raman signal and fluorescence background which will interfere with the weak tissue Raman signal. To date these technical limitations remain unsolved.

Recent technological advances, including the development of rapid Raman spectroscopy techniques and miniaturized fiber-optic Raman probes with confocal capability, have enabled real-time histopathological assessments *in vivo,* (i.e., optical biopsy), during ongoing endoscopy. Raman spectroscopy studies of colorectal polyps have tended to be limited to the so-called fingerprint (FP) spectral range (e.g., 800-1800 cm⁻¹). Some attentions have been directed towards the use of a high-wavenumber (HW) regime (e.g., 2800-3600 cm⁻¹) since this spectral range exhibits stronger tissue Raman signals as well as less interferences from the silica background from fiber-optic Raman probes. Documents WO2014/129970 A and US2013231573 A1 disclose examples of such techniques.

At present, however, there are no techniques which enable identification of cancerous cells *in vivo* that have sufficient accuracy to make this type of technique a viable option. A need exists for Raman spectroscopic techniques that enable higher accuracy identification of cancerous cells *in vivo* and *ex vivo.*

An object of embodiments in accordance with the present disclosure is to overcome at least some of the problems associated with the prior art and current endoscopic techniques for characterizing, identifying, and/or diagnosing the type and/or nature of tissue(s) such as abnormal growths, for instance, as cancers and the like in essentially any part of the body. A further object is to provide an enhanced accuracy system and method based on Raman spectroscopy for rapidly characterizing, identifying, and/or diagnosing the type or nature of abnormal tissues, such as polyps and pre-cancer, *in vivo* during endoscopic investigations (e.g., gastrointestinal endoscopic investigations such as colonoscopy).

### Summary of the invention

According to the present invention a Raman spectroscopy apparatus according to appended claim 1 and a Raman spectroscopy method according to appended claim 8 are provided.

Various embodiments in accordance with the present disclosure describe a system and method for Fiber-optic Raman spectroscopy, which provides a label-free vibrational spectroscopic technique enabling enhanced accuracy optical biopsy at the bio-molecular level *in vivo.* Multiple embodiments enable a combination of simultaneous measurements from both FP and HW spectral ranges during ongoing endoscopy. The rationale for combining the FP and HW spectral ranges for *in vivo* and *ex vivo* Raman measurements are diverse:
(i) For tissues that could exhibit intense auto-fluorescence (e.g., gastric, lung, colon, liver) which overwhelm the tissue Raman signals in the FP range, the HW range can still contain intense tissue Raman peaks with diagnostic information.
(ii) The FP and HW ranges contain complementary bio-molecular information (e.g., of proteins, lipids, DNA and water) and can therefore improve tissue characterization and diagnosis.
(iii) Different bonds vibrate in different spectral ranges, so using the two different spectral ranges (FP and HW) increases the bio-molecular information obtained in a single scan.

In accordance with an embodiment of the present disclosure, a combined FP and HW fiber-optic confocal Raman spectroscopy technique (e.g., involving the simultaneous acquisition of FP and HW spectra) can improve the real-time diagnosis of cancer, pre-cancer, and/or other abnormal growths *in vivo* during examinations of the body. The combined FP and HW technique can also be used *ex vivo* on tissue samples, to more accurately identify the types of abnormal growth present in the samples from any part of the body.

In accordance with an aspect of the present disclosure, a Raman spectroscopy apparatus includes: a first illumination source configured for directing illumination into a tissue; a Raman spectrograph configured for simultaneously detecting FP and HW Raman spectra from illumination scattered by the tissue; and a computerized control and analysis module comprising at least one processing unit and a memory storing program instructions executable by the at least one processing unit for analyzing discrete spectral sub-intervals (e.g., approximately 3 -15 or about 5-10 discrete spectral sub-intervals, where a given or each spectral sub-interval can have a spectral width of approximately 10 - 30 cm⁻¹ or about 20 cm⁻¹) of the detected Raman spectra in FP and HW wavelength ranges to identify a match with one or more reference markers in one or both wavelength ranges.

In multiple embodiments, the Raman spectrograph has a single broadband diffraction grating. The first illumination source includes a source of collimated illumination for generating an excitation energy to apply to the tissue, and the apparatus further includes a probe for transmitting the collimated illumination to the tissue and returning the detected Raman spectra from the tissue to the Raman spectrograph.

The one or more reference markers can include or be specific peaks in the detected Raman spectra. The computerized control and analysis module can include program instructions executable by the at least one processing unit for diagnosing an abnormal growth based upon the match.

The probe can include or be a confocal fiber-optic probe. The apparatus can further include an endoscope having an elongate shaft having an instrument channel within which the probe is carried.

The computerized control and analysis module can include program instructions executable by the at least one processing unit for dynamically adjusting a power of the collimated illumination, and/or dynamically adjusting a time to which the tissue is exposed to the collimated illumination,

The apparatus can include a calibration apparatus configured for standardizing the probe or the entire Raman apparatus with respect to at least one calibration reference.

The apparatus can include an additional illumination source configured for outputting additional illumination into the tissue; and a hot mirror filter configured for compensating for illumination interference between the illumination output by the first illumination source and the additional illumination output by the additional illumination source.

In accordance with an aspect of the present disclosure, a method performed by a Raman spectroscopy apparatus includes: directing illumination output by a first illumination source into a tissue; simultaneously detecting by way of a probe FP and HW Raman spectra from illumination scattered by the tissue; and analyzing discrete spectral sub-intervals in the detected Raman spectra (e.g., approximately 3 - 15 or about 5 - 10 discrete spectral sub-intervals, where a given or each spectral sub-interval can have a spectral width of approximately 10 - 30 cm⁻¹ or about 20 cm⁻¹) in both FP and HW wavelength ranges to identify a match with one or more reference markers in one or both wavelength ranges.

Simultaneously detecting FP and HW Raman spectra can include diffracting illumination in both FP and HW wavelength ranges using a single broadband diffraction grating.

The method can include diagnosing the nature of an abnormal growth based upon the match. The one or more reference markers can include or be specific peaks in the detected Raman spectra.

The method can further include dynamically adjusting the power of the illumination, and/or dynamically adjusting a time to which the tissue is exposed to the illumination.

The method can include performing a calibration or standardization procedure to standardize the probe or the entire Raman apparatus with respect to at least one calibration reference prior to illuminating the tissue.

The method can further include directing additional illumination into the tissue using an additional illumination source while directing the illumination output by first illumination source into the tissue; and compensating for illumination interference between the illumination output by the first illumination source and the additional illumination output by the additional illumination source using a hot mirror filter.

Reference will now be made, by way of example, to the accompanying drawings to provide a better understanding of embodiments in accordance with the present disclosure. The drawings should not be interpreted to be limitative and dimensions may not be to scale.
- Fig. 1: is a schematic diagram of a confocal Raman spectroscopy system for *in vivo* tissue diagnosis and characterization, in accordance with an embodiment of the disclosure;
- Fig. 2: is a block diagram of a broadband fiber-optic confocal Raman spectroscopy system developed for improved or enhanced accuracy tissue diagnosis and characterization during endoscopy, in accordance with an embodiment of the disclosure;
- Fig. 3: is a graph of broadband FP and HW *in vivo* Raman and concomitant auto-fluorescence spectra acquired from gastrointestinal (GI) mucosa in real-time, in accordance with an embodiment of the disclosure;
- Fig. 4: is a graph showing an example of interval selection applied to broadband *in vivo* Raman and concomitant auto fluorescence spectra comprising both fingerprint (FP) and high wavenumber (HW) regions, in accordance with an embodiment of the disclosure;
- Fig. 5: is a graph showing an example of interval selection applied to broadband *in vivo* Raman spectra after auto-fluorescence background subtraction in the region (a) 800-1800 cm⁻¹ and (b) 2800-3100 cm⁻¹ respectively, in accordance with an embodiment of the disclosure;
- Fig. 6: is a graph showing the Raman spectra plus or minus one standard deviation of three lesions and associated photographs of the three lesions, in accordance with an embodiment of the disclosure;
- Fig. 7A: is a graph of a difference spectra resolving spectral features, in accordance with an embodiment of the disclosure;
- Fig. 7B: is an analysis of variance (ANOVA) of three tissue categories over an entire spectral range, in accordance with an embodiment of the present disclosure;
- Fig. 7C: is a histogram of significant Raman peaks, in accordance with an embodiment of the present disclosure;
- Fig. 8A-D: are stained histopathology slides corresponding to different colorectal tissue types, in accordance with known practices;
- Fig. 9A: is a posterior probability representation, in accordance with an embodiment of the present disclosure;
- Fig. 9B: is a graph of receiver operating characteristics (ROC) for diagnosing adenoma and adenocarcinoma, in accordance with an embodiment of the present disclosure;
- Fig. 10: is a graph of the receiver operating characteristics for distinguishing adenoma from benign polyps, in accordance with an embodiment of the present disclosure;
- Fig. 11A: illustrates mean *in vivo* FP/HW Raman spectra ± 1 standard deviation of a training dataset (80% of a total dataset) for diagnostic algorithm development for Esophageal Squamous Cell Carcinoma (ESCC), in accordance with an embodiment of the present disclosure;
- Fig. 11B: illustrates Difference spectra (ESCC - normal) ± 1 standard deviation resolving unique spectral features of ESCC, and corresponding images of the WLR-guided FP/HW Raman procedures on normal esophagus and ESCC in accordance with an embodiment of the present disclosure;
- Fig. 12A: shows unpaired two-sided Student's t-test on Raman peak intensities of a training dataset (80% of the total dataset) (normal (n=736); ESCC (n=202)) over an entire spectral range (i.e., 800-1800 cm⁻¹ and 2800-3600 cm⁻¹), where multiple (e.g., seven) Raman spectra sub-regions containing the most relevant diagnostic information were identified in accordance with an embodiment of the present disclosure;
- Fig. 12B: shows a histogram ± 1 SD of the most diagnostically significant Raman peaks (* p<1E-10) in accordance with an embodiment of the present disclosure;
- Fig. 13A: shows representative hematoxylin and eosin stained histopathologic slides corresponding to normal superficial keratinized squamous epithelium and the basal layer;
- Fig. 13B: shows representative hematoxylin and eosin stained histopathologic slides corresponding to invasive esophageal squamous cell carcinoma showing prominent architectural and cytological atypia;
- Fig, 14A-14C: show posterior probabilities of *in vivo* Raman spectra belonging to (i) normal esophagus (n=736), and (ii) ESCC (n=202) of the training dataset (80% of the total dataset), using partial least square-discriminant analysis and leave-one-patient out cross validation based on the FP, HW and integrated or simultaneous FP/HW Raman techniques, respectively ((○) normal, (▲) ESCC), in accordance with an embodiment of the present disclosure;

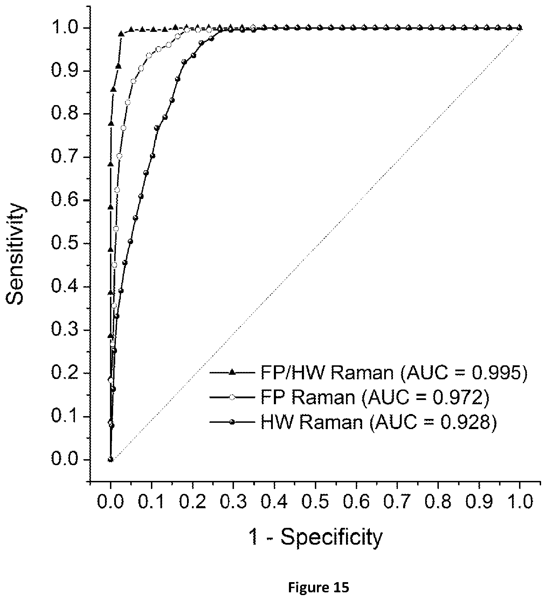

- Fig. 15: shows receiver operating characteristic (ROC) curves for separating ESCC from normal esophageal tissue for the training dataset (80% of the total dataset), where the areas under the ROC curves (AUC) are 0.972, 0.928 and 0.995, respectively, using the FP, HW and the integrated or simultaneous FP/HW Raman techniques in accordance with an embodiment of the present disclosure;

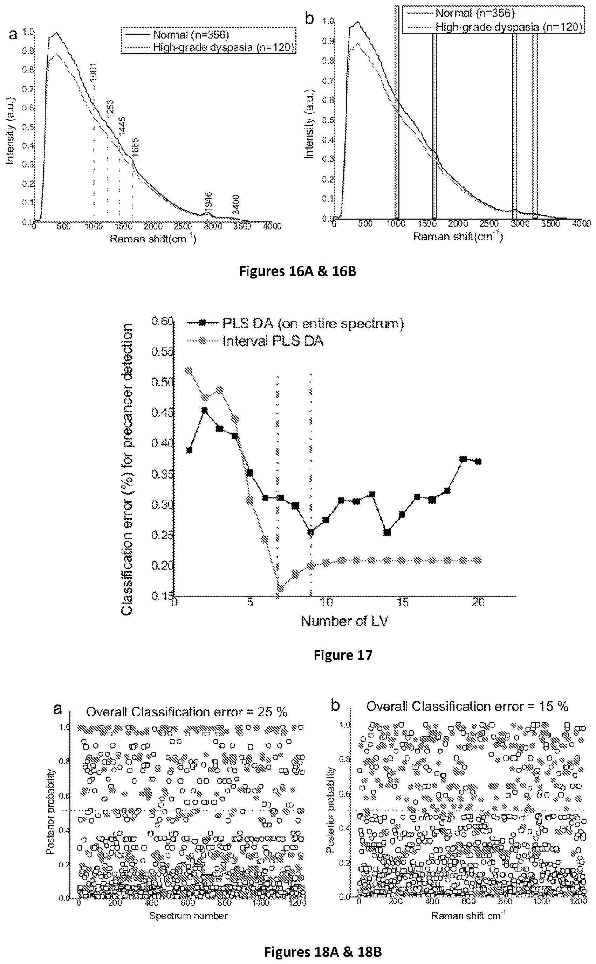

- Fig. 16A: is a graph showing a composite NIR-AF and Raman spectra measured from cervical patients, in accordance with an embodiment of the present disclosure;
- Fig. 16A: is a graph showing a composite NIR-AF and Raman spectra measured from cervical patients, in accordance with an embodiment of the present disclosure;
- Fig. 16B: is a graph showing the selected spectral regions extracted after interval PLS-DA, in accordance with an embodiment of the present disclosure;
- Fig. 17: is a graph showing a classification error plotted as a function of model complexity for PLS-DA on the entire spectrum and interval PLS-DA, which only utilizes a fraction ∼10% of the entire spectrum, in accordance with an embodiment of the present disclosure;
- Fig. 18: is a scatter plot of the posterior probabilities (normal (n=1001) and pre-cancer (n=232)) of belonging to pre-cancer group using PLS-DA modeling (a) on the entire spectrum and (b) using interval PLS-DA modeling, in accordance with an embodiment of the present disclosure;

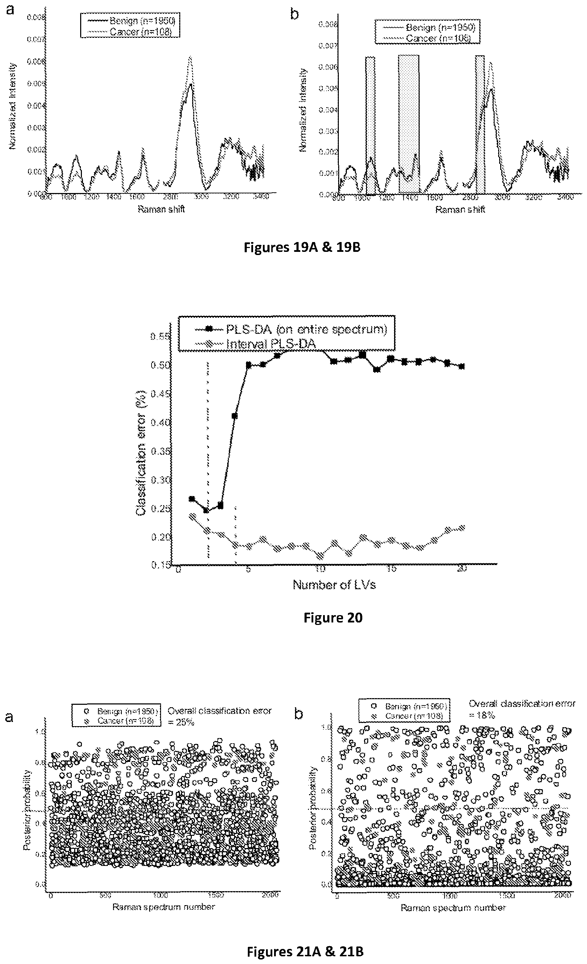

- Fig. 19: is a graph of a continuous Raman spectra and selected spectral regions after interval PLS-DA measured from 90 gastric patients (benign (n=1950) and cancer (n=108)), in accordance with an embodiment of the present disclosure;
- Fig. 20: is a graph of classification error plotted as a function of model complexity for PLS-DA on the entire spectrum and interval PLS, in accordance with an embodiment of the present disclosure;
- Fig. 21: is a scatter plot of the posterior probabilities (normal (n=1950) and cancer (n=108)) of belonging to cancer group using (a) PLS on entire FP and HW spectrum and (b) on interval PLS-DA modeling, in accordance with an embodiment of the present disclosure;

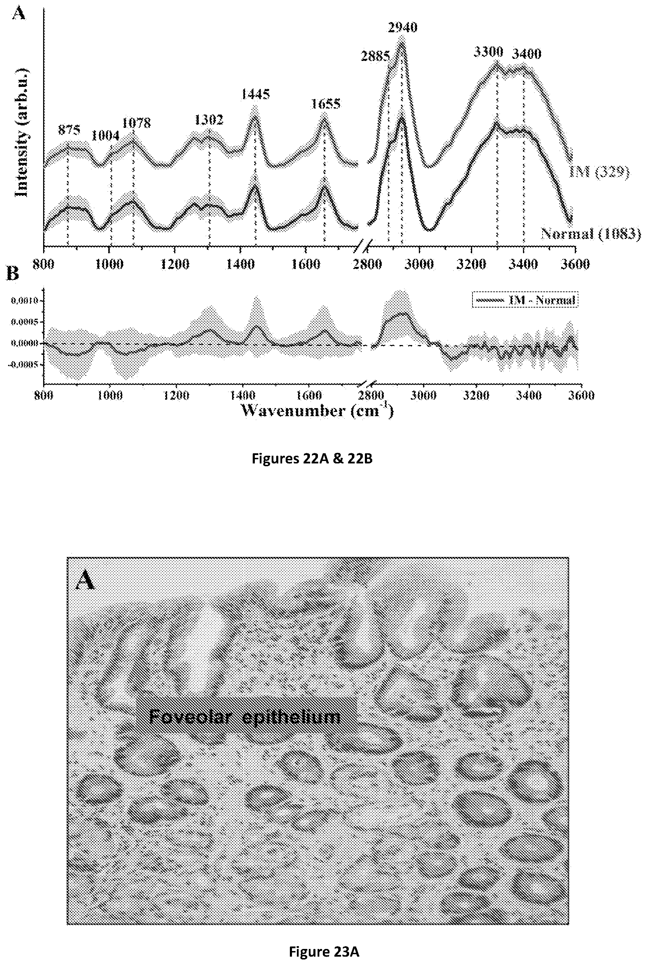

- Fig. 22A: shows mean FP and HW *in vivo* Raman spectra ± 1 standard deviation (SD) of gastric IM (n=329) and normal mucosa (n=1083) acquired from 63 patients during clinical endoscopic examination in accordance with an embodiment of the present disclosure;
- Fig. 22B: shows difference spectra (i.e., IM-normal ± 1 standard deviation (SD)) resolving the unique spectral features between normal and IM gastric tissues in accordance with an embodiment of the present disclosure;

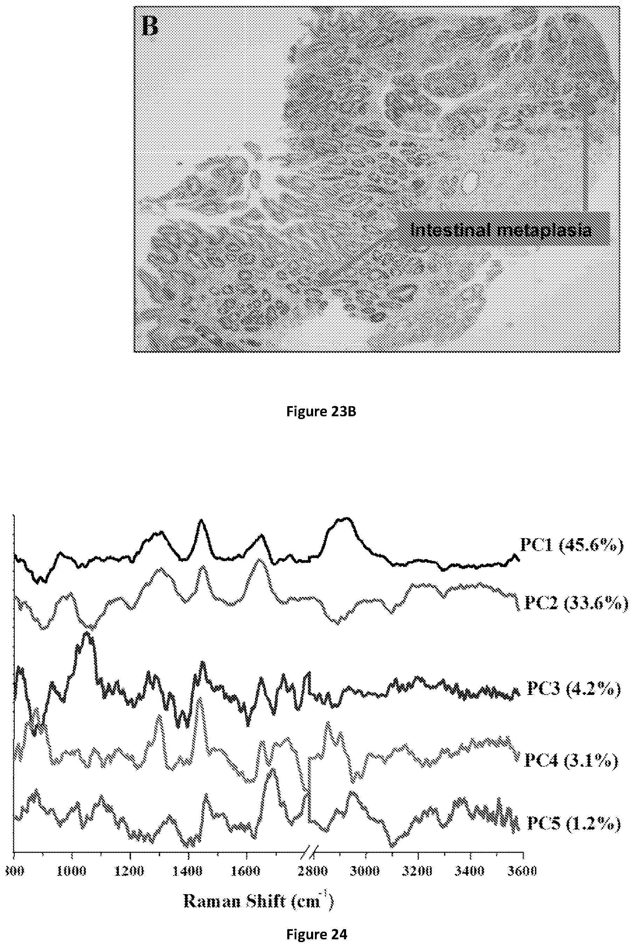

- Fig. 23A and 23B: show photomicrographs of haematoxylin and eosin (H & E)-stained sectioned slides of gastric tissues: A, normal gastric mucosa (X200 magnifications); B, extensive intestinal metaplasia (X100 magnifications);
- Fig. 24: shows the first five principal components (PCs) accounting for ∼88% of the total variance calculated from integrated FP and HW Raman spectra of gastric tissue (PC1 = 45.6%; PC2 = 33.6%; PC3 = 4.2%; PC4 = 3.1%; PC5 = 1.2%;) in accordance with an embodiment of the present disclosure;

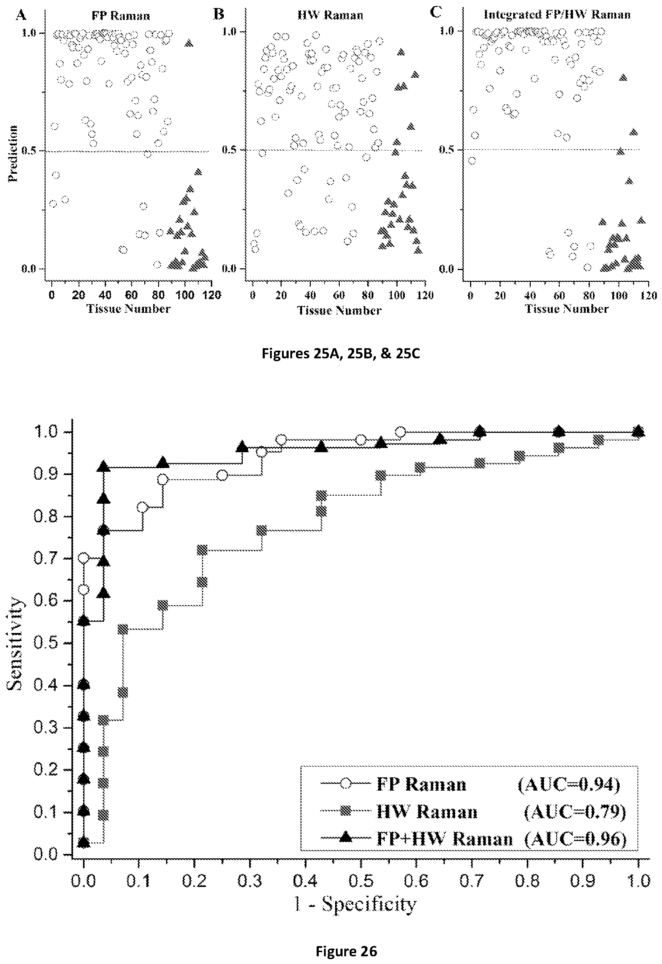

- Fig. 25A, 25B, and 25C: show scatter plots of posterior probability values belonging to normal and IM gastric tissue categories calculated by (A) FP, (B) HW and (C) integrated FP and HW Raman techniques, respectively, using PCA-LDA together with leave-one tissue site-out, cross-validation methods in accordance with an embodiment of the present disclosure, where dotted lines (0.5) give diagnostic sensitivities of 96.3% (26/27), 77.8% (21/27) and 92.6% (25/27), and specificities of 87.5% (77/88), 78.4% (69/88) and 90.9% (80/88), respectively, by using FP, HW, and the integrated FP/HW Raman techniques for separating IM from normal gastric tissue ((○) normal; (▲) IM);
- Fig. 26: shows receiver operating characteristic (ROC) curves of classification results for distinguishing IM from normal gastric tissue for the integrated FP/HW, FP, and HW Raman, respectively, together with PCA-LDA algorithms with leave-one tissue site-out, cross-validation techniques in accordance with an embodiment of the present disclosure, where integration areas under the ROC curves are 0.96, 0.94 and 0.79, respectively, for the integrated FP/HW Raman, FP Raman, and HW Raman techniques;

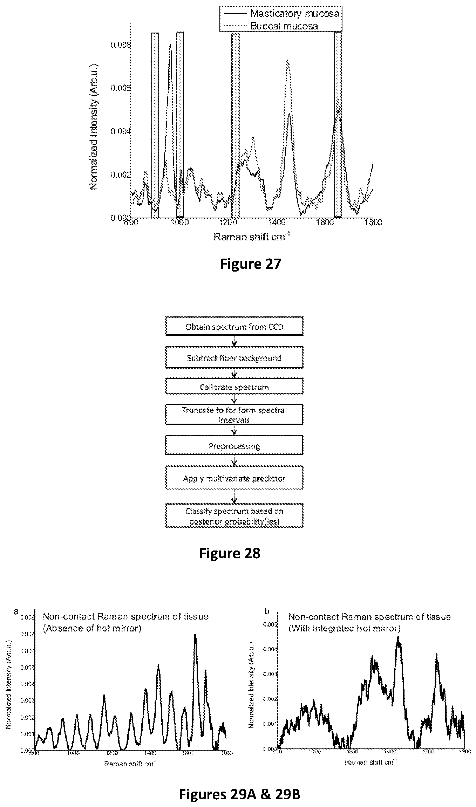

- Fig. 27: is an example of interval PLS-DA applied to Raman spectra measured from the oral cavity in which a region of large inter-anatomical variability (e.g., 956 cm⁻¹ of hydroxyapatite, 1302 cm⁻¹ and 1445 cm⁻¹ of lipids) is discarded by the variable selection techniques, in accordance with an embodiment of the present disclosure;
- Fig. 28: is a flow chart of a method of processing the Raman spectrum for prediction when discrete spectral intervals are used, in accordance with an embodiment of the present disclosure;
- Fig. 29A and 29B: show graphs of an *in vivo* Raman spectrum acquired in non-contact mode: (a) absence of a hot mirror; (b) integrated with a hot mirror, in front of a xenon light source, in accordance with an embodiment of the present disclosure;

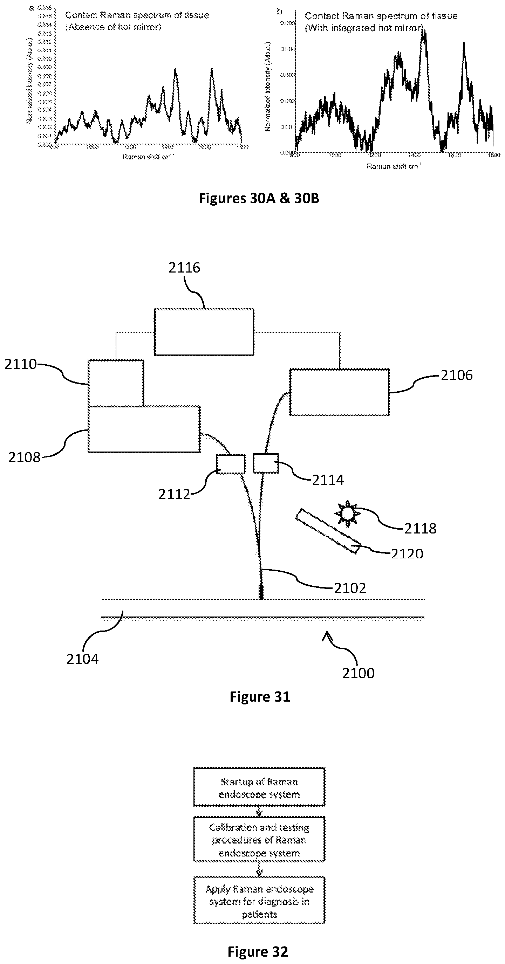

- Fig. 30A and 30B: show graphs of an *in vivo* Raman spectrum acquired in contact mode: (a) absence of a hot mirror; (b) integrated with a hot mirror, in front of a xenon light source, in accordance with an embodiment of the present disclosure;
- Fig. 31: is block diagram of a generalized system for the illumination light filtering for any application of fiber-optic Raman spectroscopy in biomedicine, in accordance with an embodiment of the present disclosure;
- Fig 32: is a flow chart of a calibration method, in accordance with an embodiment of the present disclosure;

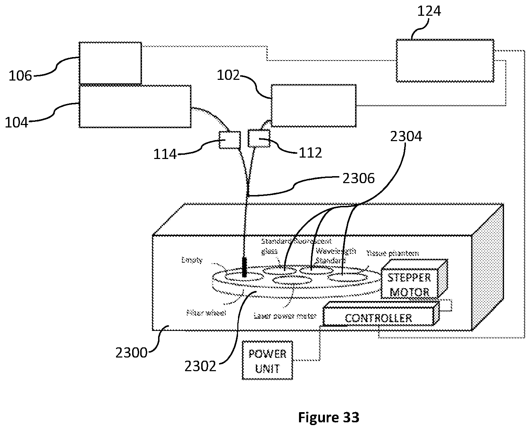

- Fig. 33: is a block diagram of a calibration device used for testing and calibrating the fiber-optic Raman endoscopy technique prior to use in patients, in accordance with an embodiment of the present disclosure;

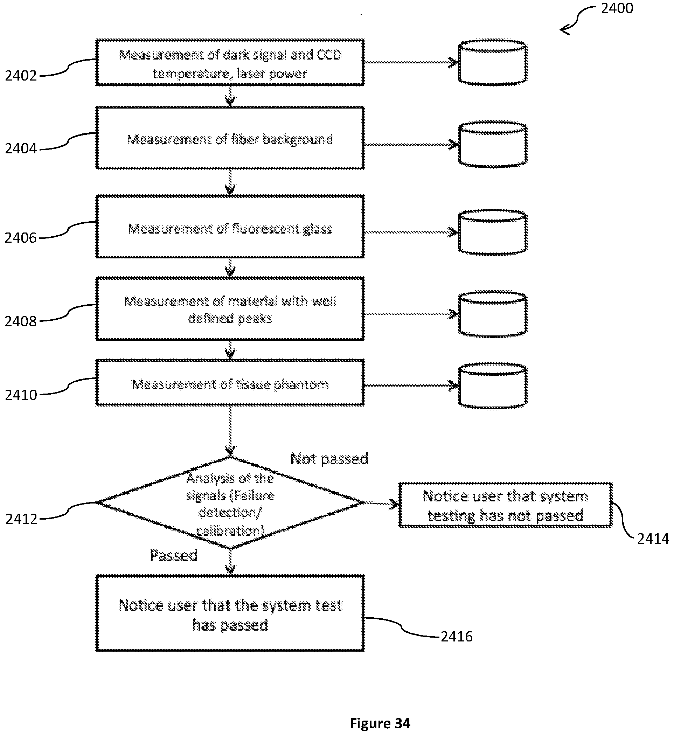

- Fig. 34: is a diagram of a testing routine for Raman endoscopy prior to use in patients, in accordance with an embodiment of the present disclosure;

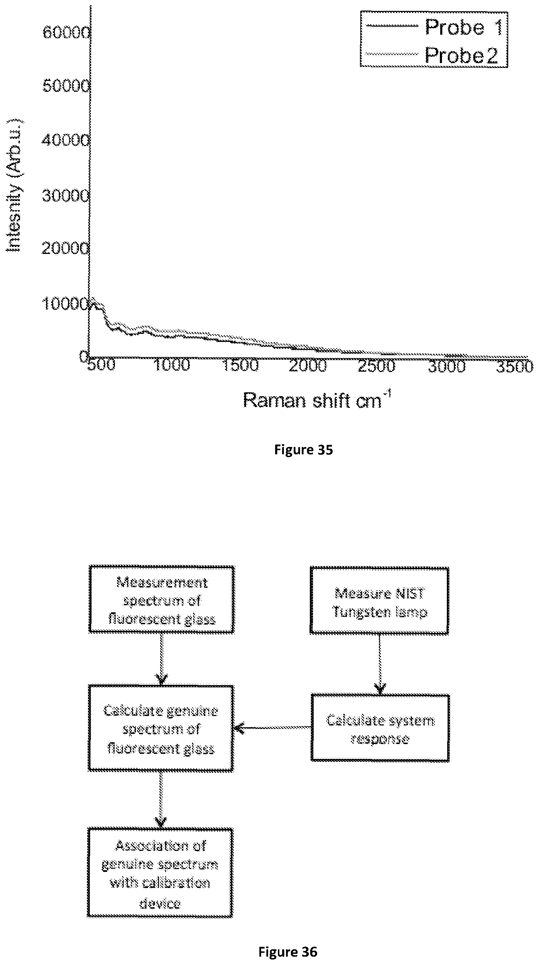

- Fig. 35: is an example graph of background spectra acquired using two different fiber-optic probes, in accordance with an embodiment of the present disclosure;
- Fig. 36: is a block diagram showing a technique or method of calibrating a fluorescence standard glass container in the calibration device using a standard tungsten lamp, in accordance with an embodiment of the present disclosure;

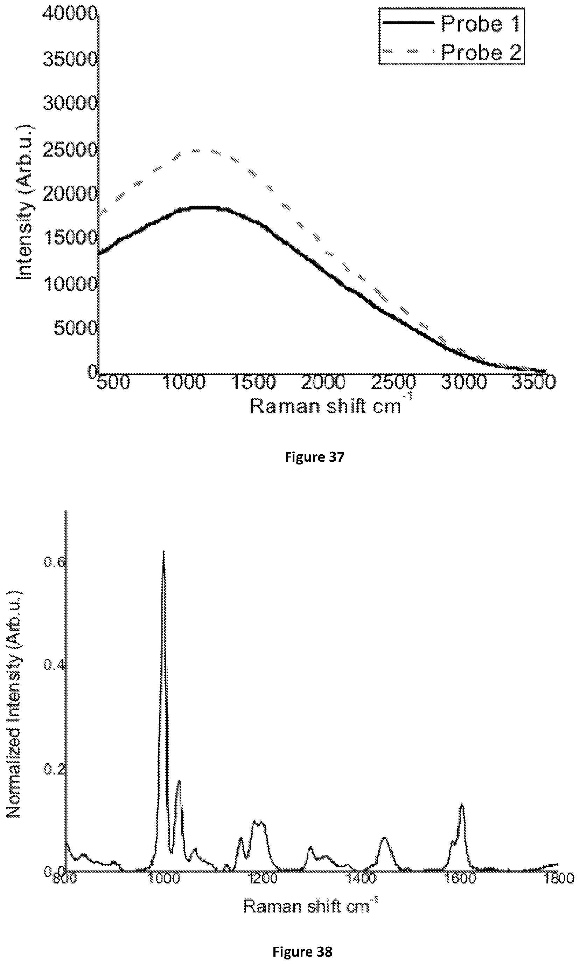

- Fig. 37: is a graph showing an example of calibration functions for two different fiber-optic probes, in accordance with an embodiment of the present disclosure;
- Fig. 38: is a graph showing an example of measuring a material with well-defined Raman peaks (e.g., polystyrene), in accordance with an embodiment of the present disclosure;

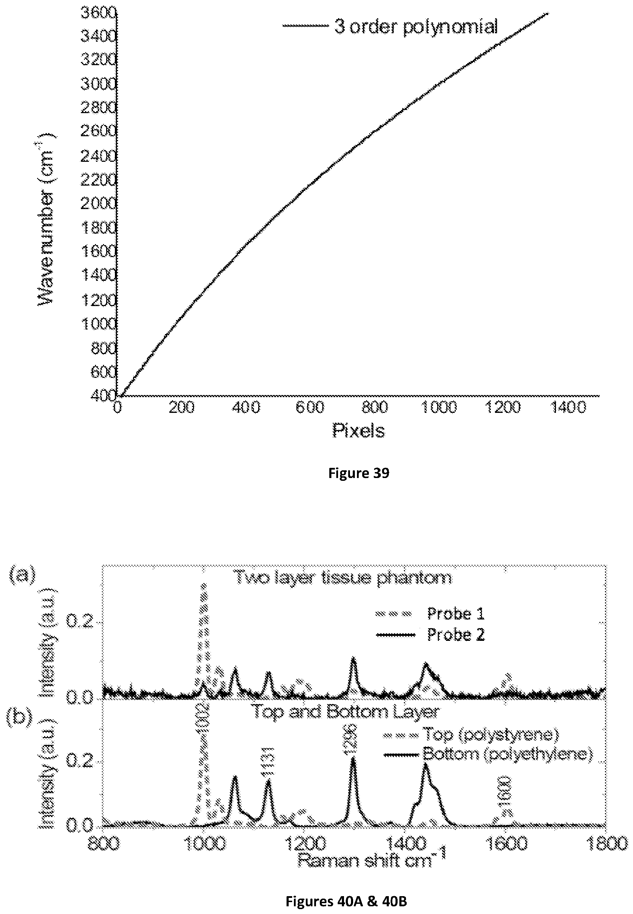

- Fig. 39: is a graph showing an example of measuring polynomial mapping of wavenumber vs. pixel number, in accordance with an embodiment of the present disclosure;
- Fig. 40A: is a graph showing a comparison of Raman spectra acquired from a two-layer tissue phantom (i.e., polystyrene and polyethylene) using 2 different Raman probes, in accordance with an embodiment of the present disclosure;
- Fig. 40B: is a graph showing a representative Raman spectra of a top layer and a bottom layer in the tissue phantom, in accordance with an embodiment of the present in disclosure;

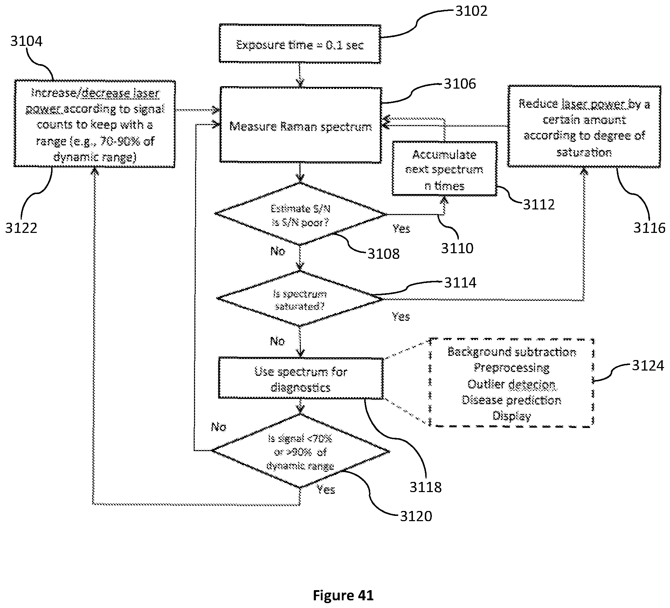

- Fig. 41: is a block diagram of a technique or method of improving S/N ratio and preventing CCD saturation by automatically adjusting laser excitation power and accumulations, in accordance with an embodiment of the present disclosure;

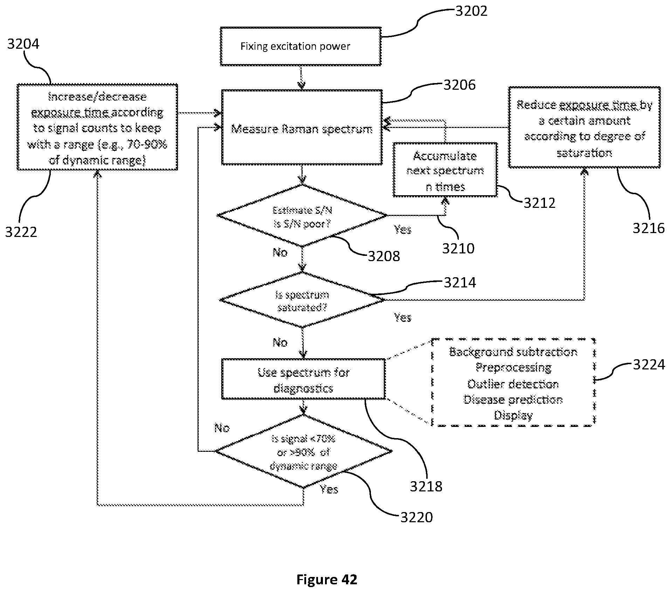

- Fig. 42: is a block diagram of a technique or method of improving S/N ratio and preventing CCD saturation by automatically adjusting exposure time and accumulations, in accordance with an embodiment of the present disclosure; and

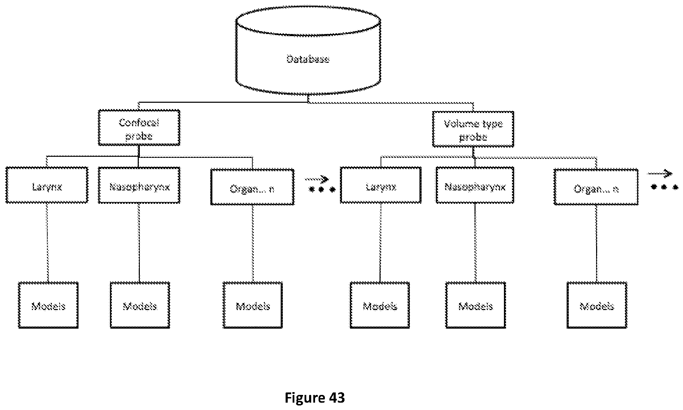

- Fig. 43: is a block diagram showing a representative Database structure for storing diagnostic models for different probes and different organs, in accordance with an embodiment of the present disclosure.

Raman spectroscopy represents a unique optical vibrational technique based on the fundamental principle of inelastic light scattering. When incident laser light induces a polarization change in molecules, a small proportion of incident photons (∼1 in 10⁸) are inelastically scattered with frequency shifts corresponding to the specific Raman active vibrational modes of the molecules in the sample. Different molecules and different bonds vibrate at different frequencies. Raman spectroscopy is therefore capable of harvesting a wealth of specific bio-molecular information from a huge number of inter- and/or intra-cellular components, such as proteins, lipids and deoxyribonucleic acids (DNA), water, etc. in tissue.

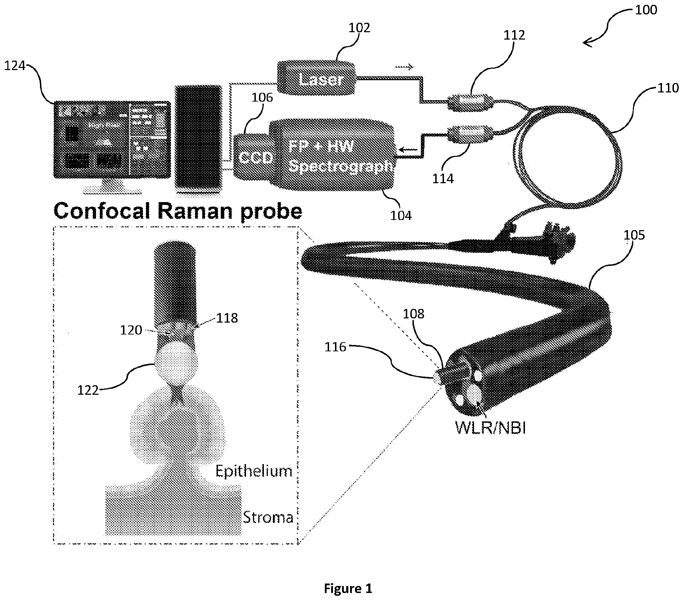

Figure 1 illustrates a fiber-optic confocal Raman spectroscopy system or apparatus, or a fiber-optic confocal Raman spectroscope 100 in accordance with an embodiment of the present disclosure. The system 100 is capable of simultaneously making Raman spectroscopy measurements in the fingerprint (FP) spectral range and the high wave number (HW) regime. Obtaining and analyzing combined FP and HW spectral range measurements in accordance with embodiments of the present disclosure enables enhanced accuracy *in vivo* and *ex vivo* analysis of bio-molecular information, for instance, for characterizing, identifying, and/or diagnosing gastrointestinal tissue(s).

The fiber-optic confocal Raman spectroscope 100 includes a collimated illumination source such as a near infrared (NIR) diode laser 102; a high-throughput reflective imaging spectrograph 104; a NIR-optimized charge-coupled device (CCD) camera 106; and a probe 108 optically coupled to both the laser 102 and the spectrograph 104 by way of an optical fiber 110. The probe 108 can be carried by an elongate shaft 105 of an endoscope (e.g., within an instrument channel provided by the elongate shaft 105). The fiber-optic Raman spectroscope 100 can further include a band pass filter 112 for background rejection of laser light illumination, and a long pass filter 114 for passing tissue Raman signals while eliminating scattered laser light and fiber background interference. A computer or microcontroller system 124 can provide an automated / computerized control and analysis module for controlling aspects of Raman spectroscope operation and performing Raman spectral analyses.

In a representative implementation, the near infrared diode laser 102 can have a maximum output of 300mW and a wavelength of 785nm, such as would be consistent with the device produced by, for example, B&W Tek Inc. The NIR laser 102 generates an excitation energy at the tip 116 of the probe 108 which can cause vibration in any species "illuminated" by the probe 108 and thereby give rise to a Raman spectrum. Other types of collimated illumination can be used to replace the diode laser 102. The spectrograph 104 can be equipped with thermo electric-cooling, for instance, to about -70 °C. The spectrograph 104 can be consistent with a device such as the Acton LS785 f/2, produced by Princeton Instruments Inc. The camera 106 can be consistent with a Pixies 400BR eXcelon as produced by Princeton Instruments Inc. In such a representative implementation, the spectroscope 100 can acquire *in vivo* Raman spectra in the spectral range of 400-3600 cm⁻¹ with a resolution of about 11 cm⁻¹. The illustrated devices are presented as representative examples and are not intended to be limitative. The atomic emission lines of mercury-argon spectral calibration lamps can be used for wavelength calibration. The lamps may be those consistent with the HG-1 and AR-1 produced by Ocean Optics, Inc., Dunedin, FL. All wavelength-calibrated spectra are corrected for the wavelength dependence of the system, using a tungsten calibration lamp such as a RS-10 as produced by EG&G Gamma Scientific, San Diego, CA.

In some embodiments, in order to measure the FP and HW spectra, the tissue Raman signals can be measured either by successively switching different laser excitation frequencies, or by using a dual-transmission grating to cover the entire spectral range i.e., (i.e., -150 to 1950 cm⁻¹; 1750 to 3600 cm⁻¹) in high resolution, as disclosed in International Patent Application No. PCT/SG2014/000063.

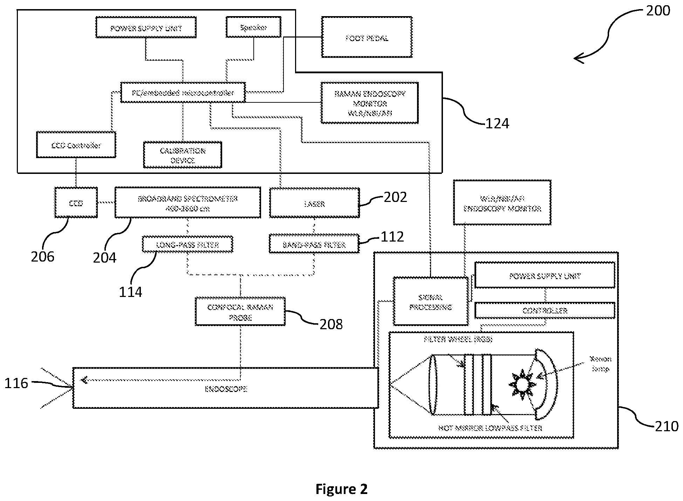

Figure 2 illustrates a further embodiment detailed herein, which is a broadband fiber-optic confocal Raman spectroscopy system or apparatus (e.g.., 400-3600 cm⁻¹) 200 that utilizes a single reflective grating for simultaneously measuring FP and HW spectra in a manner that improves the accuracy of tissue characterization and diagnosis, for instance, in endoscopically accessible organs and body parts.

The system 200 includes a near-infrared (NIR) diode laser 202 (λₑₓ = 785 nm), a high-throughput reflective spectrograph 204 equipped with a thermoelectric-cooled, NIR-optimized charge-coupled device (CCD) camera 206 and a specially designed 1.8-mm (outer diameter) fiber-optic confocal Raman probe 208. The system 200 further includes a computer / microcontroller system 124 configured for providing an automated / computerized control and analysis module for controlling aspects of Raman spectroscope operation and performing Raman spectral analyses. More particularly, the computer / microcontroller system 124 can include one or more processing units configured for executing memory-resident program instructions for performing particular Raman spectra acquisition and analysis operations, procedures, or processes in accordance with an embodiment of the present disclosure.

A customized gold-coated broadband reflective grating (e.g., 830 g/mm, having a diffraction efficiency of >90% at ∼800 nm) is incorporated or integrated into the fiber-optic confocal Raman system 200 to cover the entire spectral range (i.e., 400-3600 cm⁻¹) with a spectral resolution of ∼11 cm⁻¹. The fiber-optic confocal Raman endoscopic probe 208 is used for both laser light delivery and *in vivo* tissue Raman signal collection. The confocal Raman endoscopic probe 208 has previously been described in International Patent Application No. PCT/SG2014/000063, and includes a plurality of 200 µm filter-coated beveled collection fibers (NA = 0.22) surrounding a central light delivery fiber (200 µm in diameter, NA = 0.22). A miniature 1.0 mm sapphire ball lens (NA = 1.78) is coupled to the fiber tip of the confocal probe 208 to tightly focus the excitation light onto tissue, enabling the effective Raman spectrum collection from the epithelial lining (tissue depth < 200 µm). The fiber-optic confocal Raman probe 208 can be inserted into the instrument channel of medical endoscopes and placed in gentle contact with the epithelium for *in vivo* tissue characterization and diagnosis using a broadband confocal Raman endoscopy technique in accordance with an embodiment of the present disclosure.

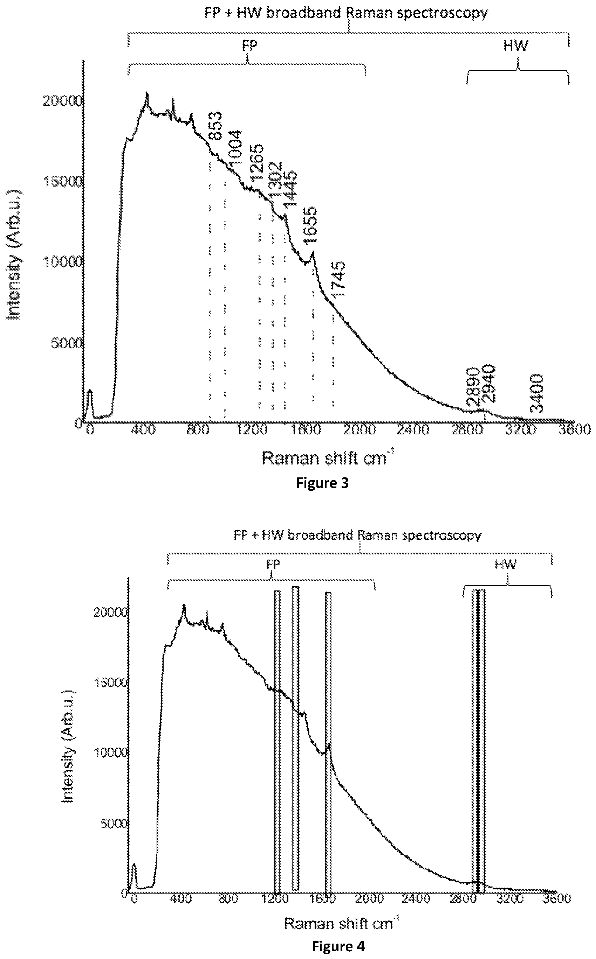

Figure 3 shows and example of broadband FP and HW Raman and concomitant auto-fluorescence spectrum measured from gastrointestinal mucosa, covering both the FP and HW regions. Highly resolved tissue Raman peaks are observed on top of the concomitant auto-fluorescence in the FP range with tentative molecular assignments as follows:
- 853 cm⁻¹ which relates to v(C-C) proteins,
- 1004 cm⁻¹ which relates to vₛ(C-C) ring breathing of phenylalanine,
- 1078 cm⁻¹ which relates to v(C-C) of lipids,
- 1265 cm⁻¹ which relates to amide III v(C-N) and δ(N-H) of proteins,
- 1302 cm⁻¹ which relates to CH₃CH₂ twisting and wagging of proteins,
- 1445 cm⁻¹ which relates to 5(CH₂) deformation of proteins and lipids,
- 1655 cm⁻¹ which relates to amide I v(C=O) of proteins, and
- 1745 cm⁻¹ which relates to v(C=O) of lipids.

Intense Raman peaks are also seen in the HW region such as:
- 2850 and 2885 cm⁻¹ which relate to symmetric and asymmetric CH₂ stretching of lipids,
- 2940 cm⁻¹ which relates to CH₃ stretching of proteins,
- 3400 cm⁻¹ in the 3100 to 3600 cm⁻¹ region which relates to the broad Raman band of water OH stretching vibrations.

This broadband technique in accordance with an embodiment of the present disclosure allows either the FP or HW range or both FP and HW spectral regions to be used simultaneously for tissue analysis, identification, characterization, and/or diagnosis, and is therefore particularly useful in endoscopically accessible organs that exhibit intense auto-fluorescence, which rapidly saturates the CCD. The computer / microcontroller system 124 can be configured for selectively using the FP spectral region, the HW spectral region, or both the FP and HW regions together for tissue analysis, identification, characterization, and/or diagnosis, for instance, in response to user input directed to a graphical user interface (GUI).

One embodiment includes the use of the HW spectral region for diagnosis if the FP region is exceeding the dynamic range of the CCD. In another embodiment, both FP and HW spectral regions are used for tissue characterization, identification, and/or diagnosis. The broadband fiber-optic confocal Raman endoscopy platform allows switching between different spectral regions (e.g., either HW, FP, or simultaneous FP and HW) according to the CCD saturation level and/or tissue type measured.

A tissue characterization, identification, or diagnosis technique or method in accordance with an embodiment of the present disclosure utilizes the complementary diagnostic information from the broadband FP and HW spectra. In general, tissue biomedical spectra are extremely complex. To convert the subtle molecular differences of Raman spectra between different tissue types into valuable diagnostic information requires sophisticated multivariate statistical analysis techniques, such as principal components analysis (PCA). This has been widely practiced by utilizing the entire (i.e., continuous) Raman spectra for tissue diagnosis and characterization on either the FP or HW regions, respectively.

PCA reduces the dimension of the Raman spectra by decomposing them into linear combinations of orthogonal components, such as principal components (PCs), such that the spectral variations in the dataset are maximized. Thus, PCA has typically been integrated with effective clustering algorithms such as support vector machines (SVM), logistic regression (LR) and linear discriminant analysis (LDA) for classification of biomedical Raman spectra. PCA is very efficient for data reduction and analysis.

Alternatively, the partial least squares (PLS)-discriminant analysis (DA) has been applied for classification problems by encoding the class membership of zeroes and ones, representing group affinities in an appropriate Y-indicator matrix. PLS-DA employs the fundamental principle of PCA, but further rotates the components, such as latent variables (LVs) by maximizing the covariance between the spectral variation and group affinity so that the LVs explain the diagnostically relevant variations rather than the most prominent variations in the spectral dataset. In most cases, this ensures that the diagnostically significant spectral variations are retained in the first few LVs.

Most multivariate algorithms (e.g., PCA or PLS-DA) are originally not designed to cope with large amounts of irrelevant spectral variables. In other words, some spectral regions in Raman spectra may have a degrading effect on the diagnostic model, for example, due to large variance, interferences, inter-anatomical variability, etc.

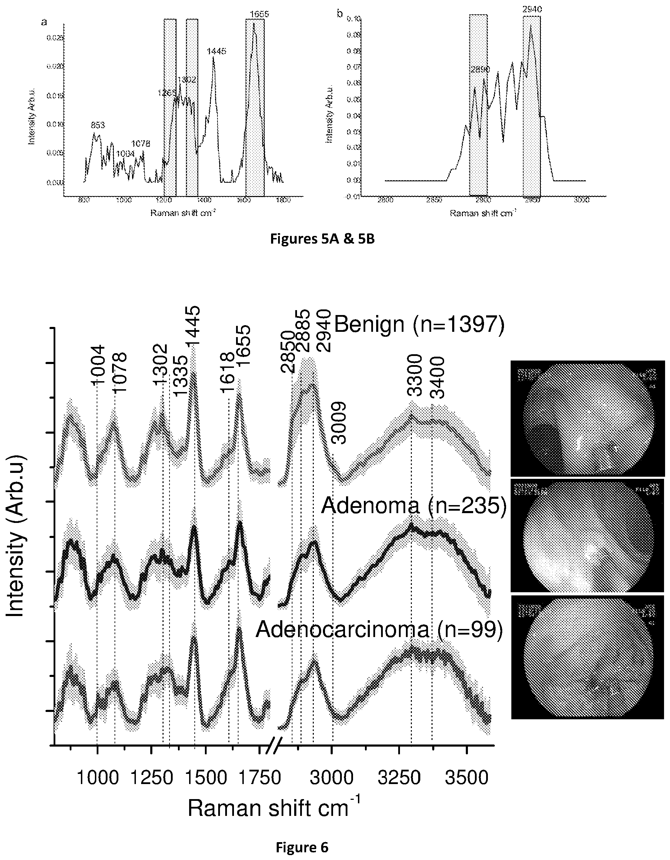

In an embodiment, a novel diagnostic technique or procedure is provided that utilizes the complementary information from the FP and HW spectral ranges, e.g., as obtained by FP and HW spectral measurements made using a broadband fiber-optic confocal Raman spectroscopy system 200. Such an embodiment makes use of discrete spectral subintervals (e.g., approximately 3 - 15 or about 5 -10 discrete spectral sub-intervals, where a given or each spectral sub-interval can have a spectral width of approximately 10 - 30 cm⁻¹ or about 20 cm⁻¹) in the broadband FP and HW Raman spectra rather than the continuous spectral ranges for diagnosis. Figure 4 shows an example from broadband Raman measurements of the gastro-intestine where complementary information has been extracted from both the FP, i.e., 800-1800 cm⁻¹, and HW, i.e., 2800-3600 cm⁻¹, spectral ranges. Figures 5A-5B show the same Raman spectra after 5^{th} order polynomial subtraction in the FP and HW range respectively, revealing the specific Raman peaks more clearly. The use of spectral sub-intervals from FP and HW region in accordance with embodiments of the present disclosure has been found to significantly improve or enhance the accuracy of *in vivo* Raman endoscopic diagnosis of abnormal growths such as pre-cancer and cancer, as will be further described below.

An embodiment in accordance with the present disclosure relates to the diagnosis of gastrointestinal abnormal growths, such as colorectal abnormal growths. The raw FP and HW Raman spectra measured from *in vivo* colorectal tissue represents a combination of weak tissue Raman signals, intense auto-fluorescence background, and noise. In order to view and analyze the Raman signals, the background and noise must be treated or removed. The raw spectra are therefore pre-processed by a first-order smoothing filter to reduce the spectral noise, such as a Savitzky Golay filter having a window width of 3 pixels. In the FP region (800-1800 cm), a fifth-order polynomial is found to be optimal for fitting the auto-fluorescence background in the noise-smoothed spectrum and this polynomial is then subtracted from the calibrated FP spectrum to yield the tissue Raman spectrum alone. In the HW range (2800-3600 cm⁻¹) a first order linear fit is found to be optimal for removing the weaker auto-fluorescence base-line. Such preprocessing of each received signal is completed within about 30 ms, and hence the processed Raman spectra and diagnostic outcomes can be displayed on a display device such as a computer screen in real-time.

Following pre-processing, the Raman spectra are analyzed to determine peaks and/or markers. These peaks or markers are then used to estimate, characterize, or determine the nature of the tissue (the nature of the lesion or abnormal growth) from which the spectra are derived. This can be done by the computer system using an appropriate means of comparison with known control spectra, reference markers, and the like. The term reference markers as used herein is intended to include one or more individual peaks in the Raman spectrum, or indeed the whole Raman spectrum. If one or more Raman reference markers are indicative of a certain nature of abnormal cell growth, a comparison between an acquired spectrum and a reference marker can be made using a lookup table or the like. It will be appreciated that many different types of comparison techniques or methods can be employed. Once the comparison has been made and a best match has been determined, the type of abnormal cell growth that is present can be indicated to the user of the system 200 by any appropriate means. This can include a visual representation on a computer screen and/or an audible message.

In a trial of an embodiment of the system 200, a total of 50 consecutive symptomatic patients were colonoscopically examined. The patients had presented for examinations for surveillance or screening of various colorectal indications such as anemia, bleeding, etc. Prior to colonoscopy, the patients were administered polyethylene glycol (PEG) electrolytes bowel preparation. Sedation was performed using intravenous administered propofol. The endoscopists cleaned the colon during inspection and prior to the confocal Raman scans, the polypoid and flat colorectal lesions were further flushed with a physiological saline solution to further reduce confounding factors (i.e., residual stool and fluid, etc.). During a typical examination the endoscope was directed to the distal colon and a Raman scan (n∼15 spectra) was performed on suspicious lesions during withdrawal of the probe from the body. Each tissue Raman measurement was acquired within a period of about 0.1 to 0.5 second. This permitted a rapid survey of colorectal polyps. Any Raman spectra that were acquired in non-contact with colonic polyps (∼10%) were automatically discarded by on-line clinical software using principal component analysis (PCA) methods associated with Hotelling's T² and Q-residual statistics. The PCA methods are the subject of previous International Patent Application No. PCT/SG2014/000063, and work as follows:
- A novel outlier detection scheme is introduced based on principal component (PCA) coupled with Hotelling's T² and Q-residual statistics to serve as a high-level model-specific feedback tool in the on-line framework. Hotelling's T² and Q-residuals are the two independent parameters providing information of within and outside the model fit.
- Using Hotelling's T² and Q-residuals parameters as indicators to control spectrum quality acquired (i.e., probe-tissue contact mode, probe handling variations, white light interference, blue light interference, confounding factors, etc.), auditory feedback has been integrated into the online Raman diagnostic system facilitating real-time spectroscopic screening and probe handling advice for the clinicians.
- If the spectra were verified for further analysis, they are fed onto probabilistic models for *in vivo* cancer diagnostics. The software can instantly switch among different pre-rendered multivariate statistical models including partial least squares-discriminant analysis (PLS-DA), PCA-linear discriminant analysis (LDA), ant colony optimization (ACO)-LDA, classification and regression trees (CART), support vector machine (SVM), adaptive boosting (AdaBoost) etc. based on a spectral databases of a large number of patients.

After the Raman scan had been completed and the results saved, each tissue specimen was removed; fixed in formalin; sectioned; stained with hematoxylin and eosin (H&E); and sent for histopathological examination. Colorectal tissues were classified into the following three clinically important categories:
(i) benign (normal and hyperplastic polyps)
(ii) adenomas (tubular, tubulovillous, villous) of low- and high-grade and,
(iii) adenocarcinomas.

The simultaneous FP and HW fiber-optic confocal Raman technique in accordance with an embodiment of the present disclosure was compared with the histopathology assessments to determine the ability to differentiate neoplastic from non-neoplastic colorectal lesions *in vivo.*

The comparison included the use of statistical analysis of the results to validate whether the simultaneous FP and HW fiber-optic confocal Raman spectroscopy technique was sufficiently accurate to replace currently used techniques. Cohen's κ statistics were calculated to assess the agreement for the histopathological characterization. Analysis of variance (ANOVA) with Fisher's *post hoc* least significant differences (LSD) test was used to test differences in means between groups. Multivariate statistical analysis was used to extract the significant Raman spectral features for clinical diagnostics. A probabilistic partial least squares (PLS) discriminant analysis (DA) was applied for tissue diagnosis. A "leave-one patient-out" cross-validation was used to assess and optimize the PLS-DA model complexity reducing the risk of over fitting. The receiver operating characteristic (ROC) curves were generated and the area under the curves (AUCs) were calculated to evaluate the capability of the FP and HW fiber-optic confocal Raman spectroscopy technique to differentiate neoplastic from non-neoplastic polyps *in vivo.*

In an experiment, which is presented by way of example only, to show the results of use of an embodiment in accordance with the present disclosure on a particular group of test subjects or patients, fifty patients (27 male and 23 female) with a mean/range age of 52/ (23-83) where enrolled for fiber-optic confocal Raman examination. Thirteen patients presented with adenomas (eleven tubular and two tubulovillous adenomas) harboring low-grade dysplasia. Three patients were associated with advanced stage colorectal adenocarcinoma. A Cohen's kappa of 0.89 demonstrated a high level of agreement between the pathological findings for the three tissue groupings. A total of 1731 *in vivo* colorectal Raman spectra were successfully acquired from 126 lesions or abnormal growths. Of these lesions, 1397 were benign, 235 were adenoma and 99 were adenocarcinoma. This was confirmed by histopathology examinations.

The detailed distribution of the patients and lesions including pathological subtypes and anatomical locations, such as ascending, transverse, descending, sigmoid, rectum, are summarized in Table 1.

**Table 1. Summary of patient statistics and details of the in vivo FP + HW confocal Raman spectra measured from different colorectal locations during clinical endoscopy.**

| | **Patients information** | | | | **Anatomical location & Raman spectra measured** | | | | | |
|---|---|---|---|---|---|---|---|---|---|---|
| **Histopathology** | **Patients** | **Lesions** | **Gender Male/female** | **Mean age** | **Rectum** | **Sigmoid** | **Descending colon** | **Transverse colon** | **Ascending colon** | **Total spectra** |
| Normal flat lesions | 28 | 91 | 13/15 | 51 | 423 | 215 | 133 | 318 | 206 | 1295 |
| Hyperplastic polyps | 5 | 7 | 3/2 | 51 | 63 | 7 | 32 | 0 | 0 | 102 |
| Adenoma | | | | | | | | | | |
| Tubular | 11 | 17 | 5/6 | 60 | 83 | 0 | 12 | 41 | 81 | 216 |
| Tubulovillous | 2 | 2 | 2/0 | 41 | 19 | 0 | 0 | 0 | 0 | 19 |
| Adenocarcinoma | 3 | 9 | 3/0 | 69 | 3 | 29 | 0 | 67 | 0 | 99 |

Figure 6 shows the mean *in vivo* confocal Raman spectra ± 1 standard deviation (SD) (shaded light-grey) measured for the benign, adenoma and adenocarcinoma abnormal growths. Prominent tissue Raman peaks with tentative assignments can be observed in the FP range at around:
- 853 cm⁻¹ which relates to *v*(C-C) proteins,
- 1004 cm⁻¹ which relates to *vₛ*(C-C) ring breathing of phenylalanine,
- 1078 cm⁻¹ which relates to v(C-C) of lipids,
- 1265 cm⁻¹ which relates to amide III *v*(C-N) and δ(N-H) of proteins,
- 1302 cm⁻¹ which relates to CH₂ twisting and wagging of lipids,
- 1445 cm⁻¹ which relates to 5(CH₂) deformation of proteins and lipids,
- 1618 cm⁻¹ which relates to *v*(C=C) of porphyrins,
- 1655 cm⁻¹ which relates to amide I *v*(C=O) of proteins, and
- 1745 cm⁻¹ which relates to *v*(C=O) of lipids,

Intense Raman peaks are also seen in the HW region such as:
- 2850 and 2885 cm⁻¹ which relate to symmetric and asymmetric CH₂ stretching of lipids respectively,
- 2940 cm⁻¹ which relates to CH₃ stretching of proteins,
- 3009 cm⁻¹ which relates to asymmetric =CH stretching of proteins,
- ∼3300 cm⁻¹ which relates to Amide A (NH stretching of proteins) as well as
- ∼3200 and ∼3400 cm⁻¹ the broad Raman band of water which relates to OH stretching vibrations that are directly related to the local conformation and interactions of OH-bonds in the cellular and extracellular space of tissue.

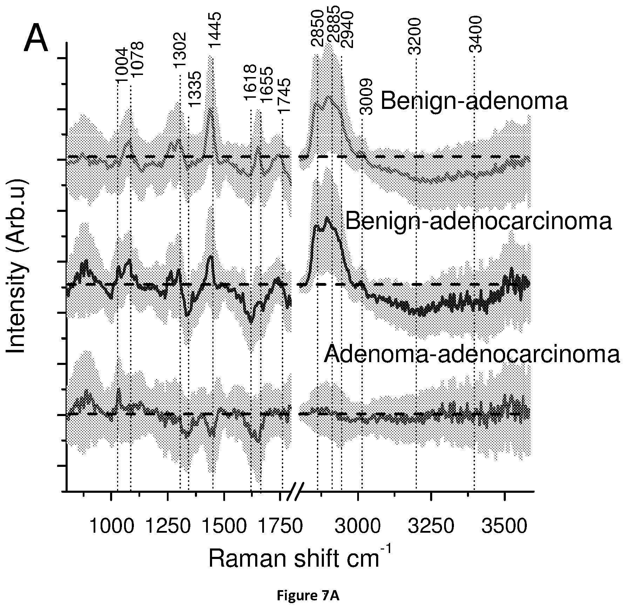

Figure 7A shows the difference spectra for benign-adenoma, benign-adenocarcinoma, and adenoma-adenocarcinoma ± 1 SD (shaded light-grey) resolving the distinctive spectral features such as peak intensity, shifting and band broadening, etc. among different colorectal lesions.

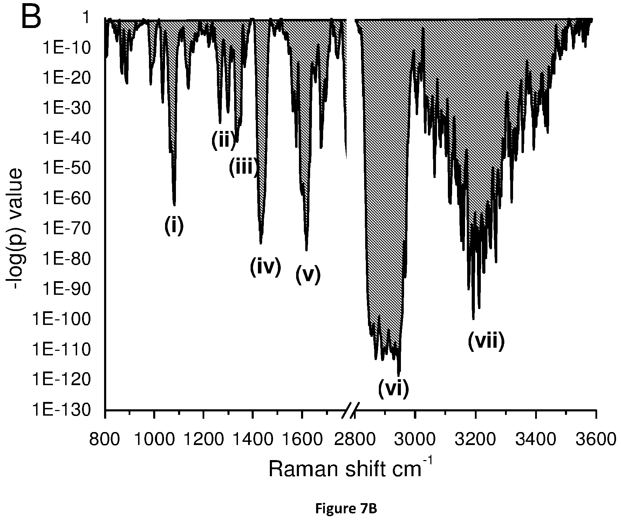

Figure 7B shows a logarithmic plot of the calculated p-values (ANOVA) for each of the Raman intensities in the entire spectral range (each Raman spectrum ranging from 800-1800 cm⁻¹ and 2800-3600 cm⁻¹ with a set of 779 intensities).

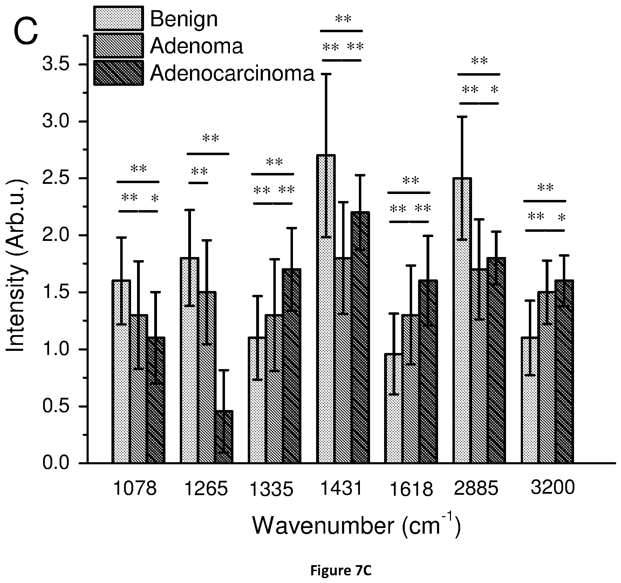

Figure 7C shows a histogram of the most diagnostically significant Raman peak intensities (mean ± 1 SD) from both the FP and HW ranges at around (i) 1078 cm⁻¹ (ii) 1265 cm⁻¹ (iii) 1335 cm⁻¹ (iv) 1431 cm⁻¹ (v) 1618 cm⁻¹ (vi) 2885 cm⁻¹ and (vii) 3200 cm⁻¹.

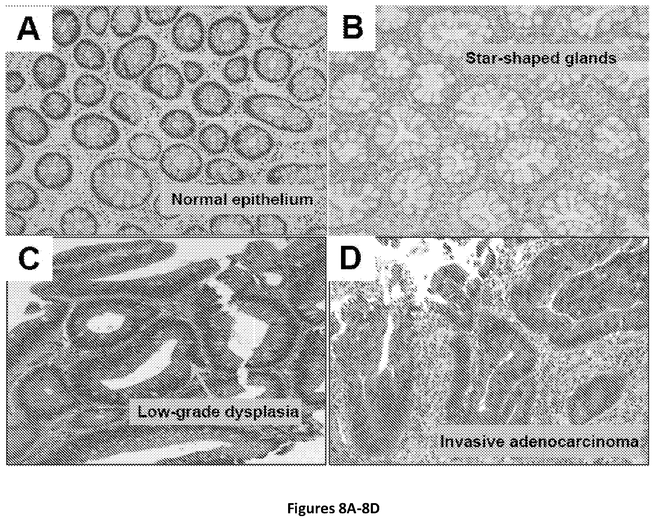

The *in vivo* Raman spectra were correlated with representative histopathology slides of the different pathology categories. Figure 8A shows the extensive presence of goblet cells in normal intestinal type mucosa with mild lymphoplasmacytic infiltrate. Figure 8B shows polypoid features with star shaped glands in a goblet type hyperplastic polyp. Figure 8C shows tubular adenoma with low-grade dysplasia illustrating crowded cells with hyper-chromatic nuclei. Figure 8D shows invasive adenocarcinoma with prominent cellular and architectural anomalies. The histopathology characterizations in Figures 8A-D reveal the cellular and morphological features of different lesion types while the FP and HW fiber-optic confocal Raman spectroscopy technique uncovers the bio-molecular changes occurring in the epithelium associated with colorectal carcinogenesis shown in Figures 7A-C.

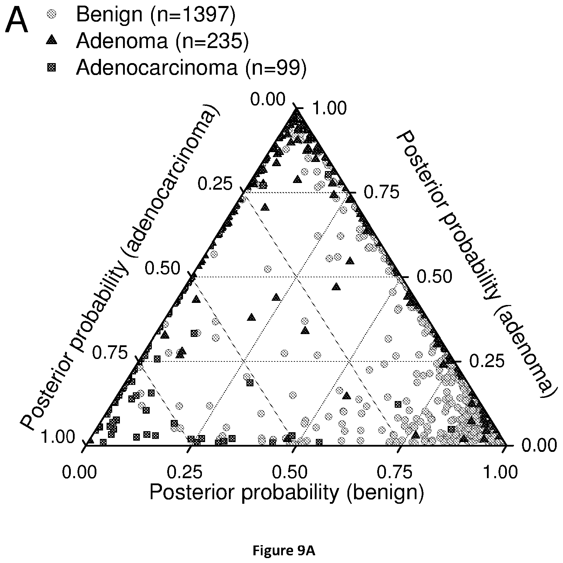

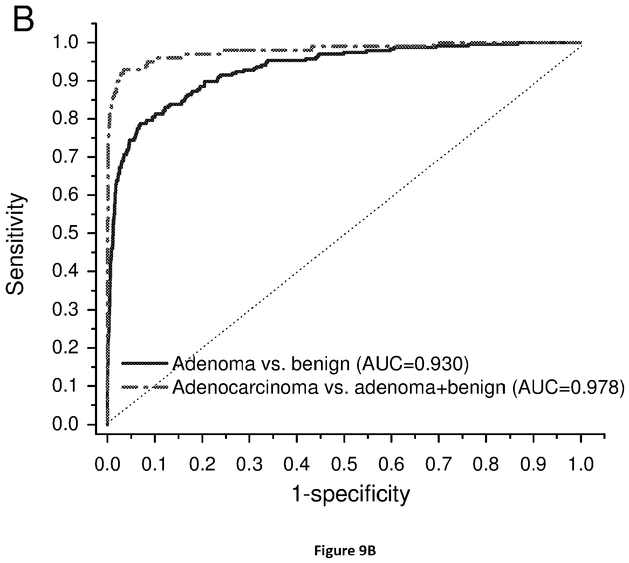

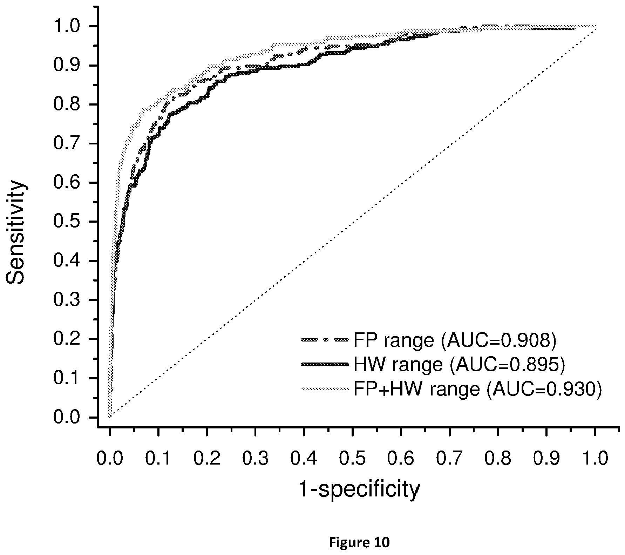

Using the complementary bio-molecular information from both the FP and HW spectral ranges, fiber-optic Raman spectroscopy for *in vivo* diagnosis is applied. Figure 9A shows the posterior probabilities generated using trichotomous probabilistic PLS-DA from the 50 colorectal patients belonging to (i) benign (n=1397), (ii) adenoma (n=235), and (iii) adenocarcinoma (n=99), respectively, using the FP and HW fiber-optic confocal Raman spectroscopy technique in accordance with an embodiment of the present disclosure. The relationships between sensitivities and specificities were determined through the development of ROC curves as shown in Figure 9B. The areas under the curves (AUC) are 0.930 and 0.978 for distinguishing adenoma from benign polyps and adenocarcinoma from adenoma and benign polyps respectively. The ROC analysis shows that adenoma could be distinguished from benign polypoid and flat lesions with a sensitivity of 88.5% (208/235) and specificity of 80.0% (1118/1397). Adenocarcinomas were detected with a sensitivity of 92.9% (92/99) and a specificity of 96.51% (1575/1632) on spectrum basis. An additional ROC analysis was conducted, as shown in Figure 10, to examine whether the bio-molecular information from the FP and HW regimes were complementary for diagnosis. The AUC was 0.908 based on the FP range (i.e., 800-1800 cm⁻¹) and 0.895 based on the HW range (i.e., 2800-3600 cm⁻¹). On the other hand, by using the complementary information from both the FP and HW ranges the robustness was significantly improved giving an AUC of 0.930. In the light of these encouraging *in vivo* results, the unique FP and HW fiber-optic confocal Raman spectroscopy technique represents a highly potent optical modality enabling real-time objective diagnosis of colorectal neoplasia *in vivo.*

It is known that a large group of people harbor small colorectal hyperplastic polyps or flat polypoid lesions that significantly add to the cost of histopathological assessments. The most significant motivation for developing and adopting advanced endoscopic modalities for colorectal examinations is the capability of discriminating benign hyperplastic polyps from adenoma. Embodiments in accordance with the present disclosure demonstrate that for the first time high quality *in vivo* confocal Raman spectra covering the FP and HW spectral ranges can be measured (e.g., simultaneously) from colorectal polyps and analyzed in real-time and this can be used to offer an improved manner of identifying neoplasia as is shown in Figure 6. The fiber-optic confocal Raman spectroscopy technique in accordance with embodiments of the present disclosure uncovers a plurality of bio-chemical and bio-molecular changes occurring in the epithelium accompanying neoplastic transformation as shown in Figures 7A-C. For instance, neoplastic polyps were associated with significant reduced Raman peak intensities at 1078 cm⁻¹ relating to *v*(C-C); 1431 cm⁻¹ relating to δ(CH₂); and 2850 and 2885 cm⁻¹ relating to symmetric and asymmetric CH₂ stretching respectively (p < 0.001) pointing to a relative reduction in lipid content. The results also show a significant up-regulated protein as indicated by the sensitive biomarkers at 1004 cm⁻¹ relating to *vₛ*(C-C) ring breathing of phenylalanine and band broadening at 1655 cm⁻¹ relating to amide I *v*(C=O) of proteins, pointing to increased cell proliferation in the neoplastic epithelium. The prominent Raman peak intensity in neoplastic polyps at 1618 cm⁻¹ (p<0.001) is also strongly linked with hemoglobin content associated with antigenic onset and resulting neo-vasculation.

The above mentioned peak intensities relate to vibrations of specific bonds within molecules. Each type of bond has a different peak intensity and identification of a peak intensity that can be associated with a specific bond can reveal bio-molecular information. The fact that a lesion or abnormal growth includes a specific bond or bonds and gives rise to a specific associated peak intensity can be used to differentiate different types of abnormal growth. For example, if an abnormal growth which is benign exhibits a specific peak intensity, later detection of that peak intensity can lead to the determination that in the later situation the lesion or abnormal growth is also likely to be benign.

In accordance with embodiments of the present disclosure, the link between peak intensity and bio markers can be exploited to identify the type of abnormal growth that is present. This can occur both *in vivo* and *ex vivo.*

In some results, the water content was found to be markedly different in adenoma. The increase in intensity of the broad anti-symmetric OH stretching vibration at 3200 cm⁻¹ (p<0.001) indicates that neoplastic epithelium holds an increased content of bound water which could partially be explained by the expression of aquaporins altering the water permeability thereby inducing hydration of the neoplastic cells. The increase of bound water may be interrelated to the simultaneous decrease of hydrophobic lipids. The significance of this finding was analyzed by calculating the peak intensity ratio (i.e., I₂₈₈₅/I₃₂₀₀) associated with lipid and bound water. This peak ratio alone may distinguish adenoma from benign polyps with a sensitivity of 81.3% (191/235) and a specificity of 80.4% (1132/1397). Hence, the lipid content and water perfusion in polyps are very useful biomarkers for colorectal neoplasia *in situ.* The direct correlation of the epithelial Raman spectral signatures with cell and tissue bio-chemistry can therefore deepen the understanding of colorectal carcinogenesis at the bio-molecular level *in situ.* Use of the peaks or markers can be employed to diagnose the type of abnormal growth type which is present.

By capitalizing on the broad range of complementary optical biomarkers including proteins, lipids, DNA and the conformations of protein-bound and unbound water, for the first time it has been demonstrated that accurate diagnosis of adenoma can be realized *in vivo* as shown in Figures 9A-B. Adenoma could be distinguished from benign polypoid and flat lesions with a sensitivity of 88.5% (208/235) and a specificity of 80.0% (1118/1397) on spectrum basis. Although the fiber-optic confocal-type Raman probe selectively interrogates the shallow epithelial layer it was still able to efficiently discriminate adenocarcinomas from adenomas signifying that invasive cancer cells are associated with prominent bio-molecular abnormalities.

The effectiveness of FP and HW fiber-optic confocal Raman colonoscopy technique for *in vivo* detection and diagnosis of colorectal neoplasia is clear from the results. It has also been demonstrated that by utilizing both the FP and HW spectral ranges yields an AUC that was superior to using either of the FP or HW ranges alone. These substantial results establish that the FP and HW Raman spectroscopy technique can largely reduce the misclassification rate, confirming the addition of complementary bio-molecular information from the FP and HW ranges for enhancing the colorectal diagnosis *in vivo.* For instance, the HW spectral range contains information related to local conformation of water as well as CH₂ and CH₃ stretching moieties that are not contained in the FP range. The combination of FP and HW ranges can also be used for diagnosis purposes *ex vivo.*

It should be noted that the combination of the FP and HW Raman spectroscope techniques is typically a preferred method of operation, such as by way of simultaneous FP and HW spectral measurements. However, the use of either FP or HW *in vivo* yield results which are capable of identifying different types of abnormal growth. As a result, embodiments in accordance with the present disclosure can use FP alone, HW alone, or a combination of FP and HW (e.g., simultaneously).

It should also be noted that the use of discrete spectral sub-intervals (which may also be referred to as predetermined values and/or reference markers) in the broadband FP and HW spectra provides an improved diagnosis technique or method.

The use of a single gold-coated broadband reflective grating (e.g., for obtaining FP and HW spectra simultaneously) is an important development in the equipment for enhanced accuracy target tissue characterization and diagnosis, e.g., for characterizing / diagnosing potentially abnormal or abnormal growths in vivo with increased accuracy in real time during an endoscopic procedure. The single gold-coated broadband reflective grating means that there is no need to switch between diffraction gratings during *in vivo* measurements, as had been the case in the prior art. This clearly has many advantages. A system or device in accordance with an embodiment of the present disclosure is thus able to make all necessary measurements without any switching processes being required. The device is more compact and cost-effective as it has only one grating rather than multiple gratings. Although the singe grating design is a bit compromised in spectral resolution compared to a dual-grating design, this compact system would not affect or significantly affect diagnostic purposes or outcomes as the spectral resolution of the compact system matches the tissue Raman spectral bandwidth that is usually in the range of ∼10 cm⁻¹.

Fiber-optic confocal Raman spectroscopy provides objective uninterrupted real-time computerized diagnosis, which is straightforward to operate and requires no additional endoscopic training or administration of contrast agents. By enabling functional and bio-molecular/bio-chemical assessment of the intestinal epithelium *in vivo,* the introduction of FP and HW fiber-optic confocal Raman spectroscopy will have a major impact on gastrointestinal endoscopy practice, such as colonoscopic practice. This new bio-molecular endoscopic approach enables objective and immediate decision-making during clinical colorectal examinations. There can be two key roles for fiber-optic confocal Raman spectroscopy in colorectal examinations as follows:
(i) Preventive and interventional approaches including identification of small high-risk adenomas for immediate polypectomy or EMR. Hyperplastic polyps or flat suspicious lesions that are clearly low risk in nature could be left *in situ* thereby efficiently reducing medical cost;
(ii) Fiber-optic confocal Raman spectroscopy may also be used to efficiently confirm or reject the presence of colorectal adenocarcinoma with a high degree of accuracy.

Embodiments in accordance with the present disclosure open up the possibility for use in endoscopic and laparoscopic surgery of CRC thereby offering the gastroenterologist an objective tool for real-time assessment and definition of resection margins as well as the follow up evaluation of post-treatment efficacy or recurrence at the molecular level. This can aid in complete tumor excision and subsequent margin assessment to reduce the risk of recurrence. Thus, FP and HW fiber-optic confocal Raman spectroscopy in accordance with embodiments of the present disclosure can be used in the field of gastrointestinal endoscopy, as well as other body sites, in both screening settings and therapeutic colorectal applications by enabling real-time, *in vivo* objective tissue assessments.

Another clinical example utilized an embodiment in accordance with the present disclosure for real-time *in vivo* diagnosis of esophageal squamous cell carcinoma (ESCC) during endoscopy by way of simultaneously acquiring both fingerprint (FP) (i.e., 800-1800 cm⁻¹) and high-wavenumber (HW) (i.e., 2800-3600 cm⁻¹) Raman spectra from esophageal tissue *in vivo.* In this set of experiments, a total of 1172 high-quality *in vivo* FP/HW tissue Raman spectra (normal (n=860); ESCC (n=312)) were acquired from 48 esophageal patients undergoing routine endoscopic examination. The total *in vivo* Raman dataset was split into two parts: i.e., 80% of the total dataset for training (938 *in vivo* FP/HW Raman spectra [normal (n=736); ESCC (n=202)] from 34 esophageal patients); while the remaining 20% of the total dataset for predictive testing (234 *in vivo* FP/HW Raman spectra [normal (n=124); ESCC (n=110)] from 14 esophageal patients).

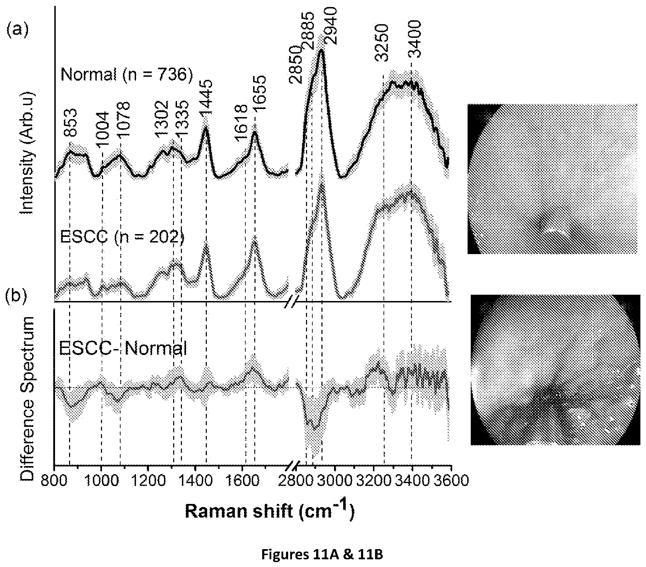

Figure 11A shows the mean *in vivo* FP/HW tissue Raman spectra ± 1 standard deviation (SD) (shaded area) of the training dataset (80% of the total dataset) for tissue diagnostic algorithms development. The corresponding images of the WLR-guided FP/HW Raman procedures are also shown. Prominent esophageal tissue Raman peaks with tentative assignments can be observed in the FP region, i.e.:
- 853 cm⁻¹ which relates to *v*(C-C) proteins,
- 1004 cm⁻¹ which relates to ring breathing of phenylalanine,
- 1078 cm⁻¹ which relates to *v*(C-C) of lipids,
- 1265 cm⁻¹ which relates to amide III v(C-N) and δ(N-H) of proteins,
- 1302 cm⁻¹ which relates to CH₂ twisting and wagging of lipids),
- 1335 cm⁻¹ which relates to CH₃CH₂ twisting of proteins and nucleic acids,
- 1445 cm⁻¹ which relates to 5(CH₂) deformation of proteins and lipids,
- 1618 cm⁻¹ which relates to v(C=C) of porphyrins,
- 1655 cm⁻¹ which relates to amide I v(C=O) of proteins, and
- 1745 cm⁻¹ which relates to v(C=O) of phospholipids.

Intense Raman peaks are also observed in the HW region as follows:
- 2580 and 2885 cm⁻¹ which relates to symmetric and asymmetric CH₂ stretching of lipids,
- 2940 cm⁻¹ which relates to CH3 stretching of proteins,
- ∼3300 cm⁻¹ which relates to amide A (NH stretching of proteins), and
- the broad Raman band of water (OH stretching vibrations peaking at ∼3250 and ∼3400 cm⁻¹) that are related to the local conformation and interactions of OH-bonds in the intracellular and extracellular space of esophageal tissue.

Figure 11B shows the difference Raman spectra between ESCC and normal esophageal tissue ± 1SD (shaded area), reflecting the Raman-active component changes associated with cancerous progression in the esophagus. The significant difference (p=1.3E-8, unpaired two-sided Student's t-test) in Raman spectra of ESCC and normal tissue discerned demonstrates the potential of simultaneous FP/HW Raman endoscopy for *in vivo* diagnosis of esophageal cancer.

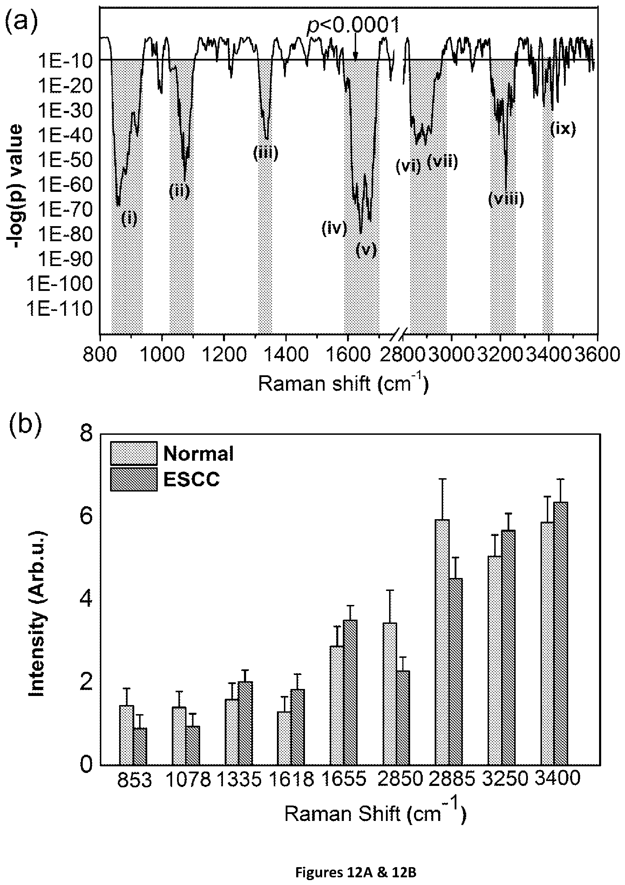

To elucidate the diagnostically important Raman-active components, Figure 12A shows a logarithmic plot of the calculated p-values (unpaired two-sided Student's t-test) for each of the Raman intensities in the entire spectral range (i.e., 800-1800 cm⁻¹ and 2800-3600 cm⁻¹). In particular, the following spectral sub-regions with statically significant difference (p<1E-10) between ESCC and normal esophagus were found: 840-940 cm⁻¹, 1025-1100 cm⁻¹, 1310-1355 cm⁻¹, 1585-1690 cm⁻¹, and 2830-2975 cm⁻¹ related to proteins, lipids and nucleic acids. Significant spectral differences were also observed in bound water in the ranges of 3160-3260 cm⁻¹ and 3370-3420 cm⁻¹.

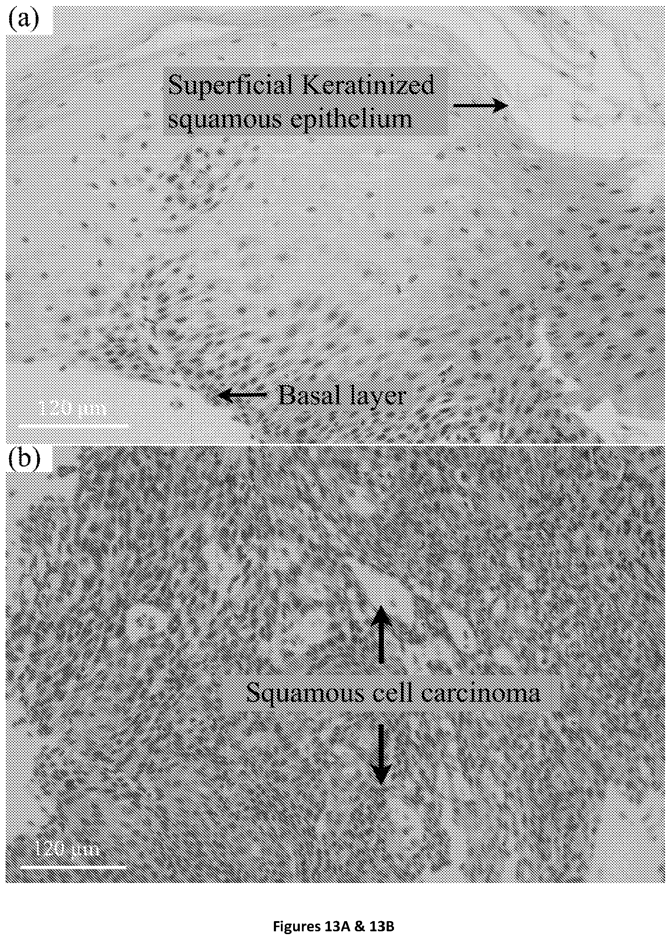

Figure 12B shows a histogram of the most statistically different Raman peak intensities (mean±1SD) for both FP and HW ranges, i.e., (i) 853 cm⁻¹, (ii) 1078 cm⁻¹, (iii) 1335 cm⁻¹, (iv) 1618 cm⁻¹, (v) 1655 cm⁻¹, (vi) 2850 cm⁻¹, (vii) 2885 cm⁻¹, (viii) 3250 cm⁻¹, and (ix) 3400 cm⁻¹. As indicated in Figure 13, the histopathology identifies prominent cellular and architectural anomalies in ESCC, the relatively higher or lower FP/HW tissue Raman bands representing different Raman-active components reveal the specific biochemical/biomolecular changes of esophageal tissue accompanied with ESCC transformation. The changes of FP/HW Raman spectra related to lipids, proteins, DNA and water contents in tissue reconfirm the capability of simultaneous FP/HW Raman spectroscopy to detect ESCC at the molecular level.

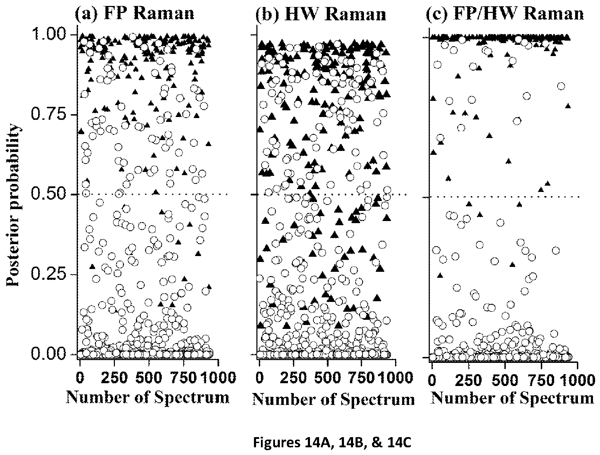

Capitalizing on the complementary biochemical/biomolecular information identified in both the FP and HW spectral ranges, PLS-DA and LOPCV were implemented on the training dataset (80% of the total dataset) to develop robust diagnostic model for enhancing *in vivo* ESCC diagnosis. A Cohen's kappa of 0.91 demonstrated a high level of agreement between the independent pathologists for the esophageal tissue groupings. Figure 14 shows the scattered plots of cross-validated PLS-DA posterior probability of each Raman prediction for (a) FP, (b) HW, and (c) integrated FP/HW, respectively. The diagnostic accuracy with integrated FP/HW Raman spectroscopy is 97.3% [sensitivity of 97.0% (196/202) and specificity of 97.4% (717/736)], superior to using either FP (accuracy-90.9%; sensitivity-93.6% (189/202), and specificity-90.2% (664/736)) or HW (accuracy-85.5%; sensitivity-78.2%(158/202), and specificity-87.5% (644/736)) Raman technique alone.

The receiver operating characteristic (ROC) curves were also generated as shown in Figure 15, with the integration areas under the ROC curves of being 0.972, 0.928 and 0.995, respectively, for the FP, HW and the integrated FP/HW techniques. The above results confirm that integrated or simultaneous FP/HW Raman technique provides the best diagnostic performance for *in vivo* ESCC detection as compared to FP or HW Raman technique alone.

In the light of these promising diagnostic results, simultaneous FP/HW Raman spectroscopy and diagnostic algorithms developed were applied for predictive diagnosis of the independent testing dataset (20% of the total dataset). The predictive accuracy of 93.2% [i.e., sensitivity-92.7% (102/110) and specificity-93.6% (116/124)] can be achieved with integrated FP/HW Raman spectroscopy, substantiating the advantages of integrated FP/HW Raman spectroscopy over either FP (predictive accuracy-91.0%; sensitivity-90.9% (100/110), and specificity-91.9% (113/124)) or HW (predictive accuracy-80.3%; sensitivity-76.4% (84/110), and specificity-83.9% (104/124)) Raman technique alone for *in vivo* ESCC.

In a further embodiment, the identification of other types of cancer were considered, for example, using the system 200 of Figure 2 to identify cervical cancers or other types of abnormal growths.
To select the complementary spectral intervals from the FP and HW spectral regions, variable/feature selection techniques are incorporated into the broadband Raman endoscopy technique. The benefits of variable/feature selection include:
1. improving the predictive performance;
2. reducing model complexity; and
3. gaining insights into the underlying spectroscopic process, such as the importance of variables/features.

In an embodiment, an interval PLS-DA algorithm is used, but in principle other, multiple, or all feature/variable selection techniques or methods can be applied to select the complementary spectral regions for any clustering algorithm, such as PCA-LDA, SVM, LR etc. The feature/variable selection techniques could be genetic algorithms (GA), swarm intelligence, selectivity ratios etc. Briefly, interval PLS-DA used herein performs a sequential, exhaustive search for the best combination of intervals in the Raman spectra. Accordingly, interval PLS-DA creates individual PLS models, each using only a subset or window of variables. If there are 200 intervals for a given spectral data set, 200 PLS-DA models are calculated (i.e., one for each interval).

A leave-one patient out cross-validation is performed for every model and the interval, which provides the highest diagnostic accuracy, is selected. This is the optimum single-interval model. If only one interval is desired, the algorithm stops at this point. If, however, more than one interval is desired (to increase information content and improve predictive performance), additional cycles can be performed. In the second cycle, the first selected interval is used in all models but is combined with each of the other remaining intervals, one at a time, when creating a new set of new PLS models. In this way, regions of complementary diagnostic value from the FP and HW range are extracted while redundant or irrelevant spectral ranges, such as regions of poor predictive power are excluded from the model.

Other spectral ranges (e.g., the so-called Raman silent range between 2000 cm⁻¹ to 2800 cm⁻¹) other than FP and HW ranges can be used in certain applications.

To demonstrate an application of this embodiment, a broadband spectra from a series of cervical patients was acquired. A total of 44 non-pregnant female patients (between 18 and 70 years of age) who underwent a colposcopy procedure due to an abnormal Pap smear were recruited studied. Prior to the *in vivo* tissue Raman spectral measurements, a 5% acetic acid solution was applied topically on the cervix for 2 min for evaluation of the color whitening in the tissue (the degree of white discoloration in the cervix is related to the grade of pre-cancer).

Confocal broadband Raman and concomitant auto-fluorescence spectra were measured by placing the fiber-optic confocal probe in gentle contact with the tissue. Figure 16A shows the mean raw composite Raman and auto-fluorescence spectra of normal (n = 356) and pre-cancerous (n = 120) cervical tissues. The normal and pre-cancerous cervix tissue spectra shows weak Raman vibrational bands on top of the auto-fluorescence near:
- ∼854 cm⁻¹ which relates to glycogen (CCH) deformation aromatic and (C-C) stretching of structural protein and collagen,
- ∼937 cm⁻¹ which relates to v(C-C) stretching of proline, valine, and glycogen,
- ∼1001 cm⁻¹ which relates to (C-C) ring breathing of phenylalanine,
- ∼1095 cm⁻¹ which relates to phospholipids and nucleic acids),
- ∼1253 cm⁻¹ which relates to amide III,
- ∼1313 cm⁻¹ which relates to CH₃CH₂ twisting mode of lipid/protein (collagen),
- ∼1445 cm⁻¹ which relates to CH₂ bending mode of proteins and lipids,
- ∼1654 cm⁻¹ which relates to amide I band - (C=O) stretching mode of proteins,
- ∼2946 cm⁻¹ which relates to proteins - CH₃ stretching, and
- ∼3400 cm⁻¹ which relates to water- (OH) stretching.

To demonstrate that the broadband Raman technique integrated with interval selection can improve the diagnosis of cervical pre-cancer a conventional PLS-DA is applied to the continuous spectrum (Figure 16A) and an interval PLS-DA is applied to extract complementary regions as described above. Figure 16B shows the selected spectral sub-regions (i.e., 1000-1020 cm⁻¹, 1640-1660 cm⁻¹, 2890-2910 cm⁻¹ and 3290-3310 cm⁻¹) after interval PLS-DA.

Figure 17 shows the classification error as function of algorithm complexity (i.e., number of LVs) for both PLS-DA and the interval PLS-DA model. Minima can be found at 9 and 5 LVs respectively. It is evident that by using those complementary spectral regions from both the FP and HW region rather than the entire spectrum, the accuracy significantly increases (classification error reduces from 25% to 15%). This example shows that by removing regions containing poor information and selecting the complementary sub-intervals from the FP and HW spectra, the accuracy for *in vivo* detection of pre-cancer can be significantly improved ∼10%. This indeed proves that the FP and HW regions contain complementary information for tissue characterization. The scatter plots in Figures 18A and 18B show the probabilistic classification outcomes. Consequently, the discrete intervals from the broadband confocal Raman endoscope technique now enables clinicians to obtain a more accurate probabilistic measure of the risk associated with suspicious lesions, thereby significantly improving the guidance of physical biopsies.

It should be noted that thresholds can be imposed on the probabilistic classifications (Figure 18A and 18B) by incorporating prior information such as family history of diseases. For example, if a patient belongs to a high-risk group, the apparatus may automatically select a threshold providing a higher sensitivity for pre-cancer or cancer. Other types of data to generate prior probabilities could be genetic, proteomic, epidemiologic, such as age, ethnicity, sex, drinking habits, etc., imaging outcome (e.g., CT, MRI etc.), symptoms etc. The option for adjusting thresholds has been integrated into the clinical Raman endoscope software that controls the spectral measurements.

In a further clinical example using the broadband confocal Raman endoscope fiber-optic confocal Raman spectra were acquired from a series of gastric patients, focusing on early diagnosis of gastric malignancies. Figure 19A shows the mean *in vivo* confocal FP and HW Raman spectra measured from 90 patients presenting with different tissue types: benign (n=1950 spectra) and cancer (n=108 spectra) as confirmed by histopathological characterization. Prominent tissue Raman peaks of protein, DNA and lipids can be observed at around:
- 936 cm⁻¹ which relates to *v*(C-C) proteins,
- 1004 cm⁻¹ which relates to *vₛ*(C-C) ring breathing of phenylalanine,
- 1078 cm⁻¹ which relates to v(C-C) of lipids,
- 1265 cm⁻¹ which relates to amide III *v*(C-N) and δ(N-H) of proteins,
- 1302 cm⁻¹ which relates to CH₂ twisting and wagging of proteins,
- 1445 cm⁻¹ which relates to 5(CH₂) deformation of proteins and lipids,
- 1618 cm⁻¹ which relates to *v*(C=C) of porphyrins,
- 1655 cm⁻¹ which relates to amide I *v*(C=O) of proteins,
- 1745 cm⁻¹ which relates to *v*(C=O) of lipids,
- ∼2946 cm⁻¹ which relates to proteins - CH₃ stretching, and
- ∼3400 cm⁻¹ which relates to water - (OH) stretching.

To demonstrate an embodiment in accordance with the present disclosure, conventional PLS-DA is applied to the continuous spectrum as well as interval PLS-DA. Figure 19B shows the selected complementary spectral regions after interval PLS-DA (i.e., ∼1050-1120 cm⁻¹, ∼1323-1490 cm⁻¹, and ∼2850-2870 cm⁻¹).

Figure 20 shows the classification error as function of algorithm complexity (i.e., number of LVs) for both PLS-DA and interval PLS-DA, Minima in classification error can be found using 5 LVs for both models. It is evident that by choosing those complementary diagnostic significant spectral regions within the FP and HW range rather than the entire spectrum, the model becomes more accurate (classification error reduces from 0.25% to 0.18%) as illustrated in the scatter plot in Figures 21A and 21B. The sensitivity increases from 72.2% to 75.9% and the specificity increases from 74.7% to 87.9% illustrating the complementary information improves diagnosis of gastric cancer.

Yet another clinical example utilized an embodiment in accordance with the present disclosure in which a simultaneous FP and HW fiber-optic Raman endoscopic technique was utilized for *in vivo* detection of gastric intestinal metaplasia (IM)-precancerous lesions during endoscopic examinations. In this example, the Raman spectroscopy system 200 included a near-infrared (NIR) diode laser (*λ*ₑₓ=785 nm) (maximum output: 300 mW, B&W TEK Inc.), a high-throughput reflective imaging spectrograph (Acton LS-785 f/2, Princeton Instruments Inc.) equipped with a gold-coated 830 gr/mm grating and a thermo electric-cooled, NIR-optimized charge-coupled device (CCD) camera (PIXIS: 400BR-eXcelon, Princeton Instruments Inc.). The system 200 acquired *in vivo* tissue Raman in the spectral range from 400-3600 cm⁻¹ with a resolution of ∼9 cm⁻¹. A 1.9 m long fiber-optic Raman probe 108 having a 1.8 mm outer diameter was utilized for both laser light delivery and *in vivo* epithelial tissue Raman signal collection. The Raman endoscopic probe 108 designed for endoscopy includes 18 × 200 µm bevelled collection fibers (NA=0.22) surrounding a central light delivery fiber (200 µm in diameter, NA=0.22). A 1.0 mm sapphire ball lens (NA=1.78) is coupled to the fiber tip of the probe to tightly focus the excitation light onto the gastric tissue surface, enabling the effective Raman spectrum collection from the epithelial lining. The depth-selective capability of the fiber-optic Raman spectroscopy system 200 ensures shallower tissue interrogation (<200 µm) with microscopic probing volume (<0.02 mm²), thereby reducing the interferences and signal dilution from deeper bulky tissues, while selectively or preferentially interrogating the epithelium associated with neoplastic onset and progression. The atomic emission lines of mercury-argon spectral calibration lamps (HG-1 and AR-1, Ocean Optics, Inc., Dunedin, FL) were used for wavelength calibration. All wavelength-calibrated spectra were corrected for the wavelength dependence of the system using a tungsten calibration lamp (RS-10, EG&G Gamma Scientific, San Diego, CA). The entire FP/HW fiber-optic Raman endoscopic system 200 is controllable using a foot pedal and an intuitive software framework configured for providing feedback (e.g., auditory and/or visual probabilistic feedback) to the gastroenterologist in real-time.

Raw spectra were preprocessed by a third-order Savitzky-Golay smoothing filter (a window width of 3 pixels) to remove spectral noise. In the FP region (800-1800 cm⁻¹), a fifth-order polynomial was found to be optimal for fitting the AF background in the noise-smoothed spectrum and this polynomial was then subtracted from the measured FP spectrum to yield the FP tissue Raman spectrum alone. In the HW range (2800-3600 cm⁻¹), a first-order polynomial fit was used for removing the AF background. The FP/HW Raman spectra were normalized over the integrated area under the FP and HW ranges to allow a better comparison of the spectral shapes and relative Raman band intensities between normal and IM gastric tissue. All raw spectral data were processed on-line with software developed in the Matlab environment (Mathworks Inc., Natick, MA). Principal components analysis (PCA) and linear discriminant analysis (LDA) were implemented to develop robust diagnostic algorithms for the differentiation between normal and IM gastric tissues. Leave-one tissue site-out cross-validation was utilized to evaluate the diagnostic models developed in an unbiased manner. Multiple Raman spectra (10-15) were acquired from each tissue site within 1 second, and the majority voting strategy was applied for final classification. The diagnostic outcomes were displayable on a display device such as a computer screen in real-time. Receiver operating characteristic (ROC) curves were also generated by successively changing the thresholds to determine correct and incorrect classifications for all tissues. All spectra preprocessing and multivariate statistical analysis were performed online using scripts written in the Matlab programming environment,

A total of 1412 *in vivo* Raman spectra (i.e., normal (n=1083 spectra) and IM (n=329 spectra) were successfully acquired from 115 sites (i.e., normal (n=88 sites) and IM (n=27 sites)) as confirmed by consensus histopathology examinations.

Figure 22A shows mean *in vivo* Raman spectra ± 1 standard deviation (SD) (shaded light-grey) measured (i.e., normal and IM). Prominent tissue Raman peaks with tentative assignments are observed in the FP range at:
- 875 cm⁻¹ which relates to *v*(C-C) proteins,
- 1004 cm⁻¹ which relates to *vₛ*(C-C) ring breathing of phenylalanine,
- 1078 cm⁻¹ which relates to *v*(C-C) of lipids,
- 1302 cm⁻¹ which relates to CH₂ twisting and wagging of lipids,
- 1445 cm⁻¹ which relates to *δ*(CH₂) deformation of proteins and lipids, and
- 1655 cm⁻¹ which relates to amide I *v*(C=O) of proteins.
In addition, intense Raman peaks in the HW region are also observed at
- 2885 cm⁻¹ which relates to symmetric and asymmetric CH₂ stretching of lipids,
- 2940 cm⁻¹ which relates to CH₃ stretching of proteins,
- 3300 cm⁻¹ which relates to Amide A (NH stretching of proteins), and
- the broad Raman band of water (OH stretching vibrations peaking at ∼3400 cm⁻¹) that are related to the local conformation and interactions of OH-bonds in the cellular and extra-cellular space of tissue.

Figure 22B shows the difference spectra (i.e., IM - normal) ± 1 SD (shaded light-grey) resolving the distinctive spectral features (e.g., peak intensity, shifting and band broadening) associated with IM transformation, confirming the potential of FP/HW Raman spectroscopy for early diagnosis of IM at endoscopy.

Figures 23A-B show representative hematoxylin and eosin (H&E) slides of the corresponding tissue sites using Raman endoscopy, which include (a) normal gastric mucosa (x200 magnification); (b) extensive gastric intestinal metaplasia whereby the gastric epithelium contains apparent goblet cells (x100 magnification). Histopathology characterizations reveal the cellular and morphological features of normal and intestinal metaplasia lesions in the gastric tissue, while the simultaneous FP/HW Raman endoscopy uncovers the specific biochemical constituents (e.g., proteins, lipids and water) of the epithelial tissue at the molecular level.

To develop sophisticated multivariate diagnostic algorithms and compare tissue diagnostic performance among the three different Raman techniques (i.e., FP, HW and the integrated FP/HW), PCA-LDA together with Student's *t*-test were implemented on the in vivo tissue Raman spectra acquired to evaluate the elusive differences observed in the spectra of different tissue types. The leave-one tissue site-out, cross-validated PCA-LDA diagnostic algorithms were further developed based on the diagnostic significant PCs (*p*<0.01) as shown in Figure 24, accounting for 45.6% (PC1), 33.6% (PC2), 4.2% (PC3), 3.1% (PC4) and 1.2% (PC5) of Raman spectral variations, respectively. The features of different significant PCs are distinct; in particular, some PC features, such as the peaks, troughs, and spectral shapes in Figure 24, are similar to those of tissue Raman spectral patterns. The first significant PC accounts for the largest variance within the spectral data sets (i.e., 45.6%), whereas successive PCs describe features that contribute progressively smaller variances. All the five diagnostically significant PCs are then fed into the LDA model together with leave-one tissue-out, cross-validation technique for gastric tissue diagnosis and classification.

Figure 25 shows cross-validation classification results (posterior probabilities) between normal and IM pathologies by PCA-LDA algorithm modeling as calculated for (a) FP, (b) HW and (c) the integrated or simultaneous FP/HW Raman technique, respectively. The threshold lines (0.5) applied to the posterior probability scatter plots yield diagnostic accuracies of 89.6% (103/115), 78.3% (90/115) and 91.3% (105/115) (sensitivities of 96.3% (26/27), 77.8% (21/27), 92.6% (25/27), and specificities of 87.5% (77/88), 78.4% (69/88), 90.9% (80/88)), respectively, for the FP, HW and the integrated FP/HW Raman spectroscopic techniques. The results demonstrate that the integrated FP/HW Raman spectroscopy performs best for gastric IM diagnosis as compared to FP technique alone or HW technique alone.

Figure 26 shows ROC curves generated for the FP, HW and the integrated FP/HW Raman techniques, revealing the relationships between the diagnostic sensitivities and specificities of gastric IM identification. The integration areas under the curves (AUCs) are 0.94, 0.79, and 0.96, respectively, for the FP, HW and integrated FP/HW Raman techniques, further reconfirming that the integrated FP/HW Raman technique provides the best diagnostic performance for *in vivo* gastric IM detection. Overall, the above results demonstrate the great potential of the simultaneous FP/HW Raman spectroscopic technique developed for enhancing early diagnosis of gastric precancerous lesions *in vivo* during endoscopic examination.

Figure 27 shows a further embodiment of Raman spectra measured from the oral cavity in the FP range alone. It should be noted that there is prominent inter-anatomical variability among different tissue sites in the oral cavity (e.g., buccal and masticatory mucosa). In this embodiment the regions of large inter-anatomical variance, such as 956 cm⁻¹ of hydroxyapatite, and 1302 cm⁻¹ and 1445 cm⁻¹ of lipids are excluded using the interval selection method. Hence variable/feature selection techniques can be used to reduce the effect of inter-anatomical variability on diagnostic algorithms by excluding spectral regions with large variance. This is an important point and will have applications in other organs, including skin, oral cavity etc.

Figure 28 shows an example of how the prediction can be applied prospectively in real-time to new spectra after implementation of a probabilistic diagnostic model based on distinct spectral sub-regions.

This includes several steps including fiber background subtraction calibration, truncation to the spectral intervals, preprocessing and finally application of predictor for disease classification.

This embodiment has several major advantages over the prior art: Instead of using the entire broadband spectrum for diagnosis this technique or method only uses a subset (e.g., 5-20%) of complementary information greatly simplifying the diagnostic model. Secondly the variable selection provides qualitative insights into the biochemical and bio-molecular foundation of the disease. Thirdly, the accuracy is increased significantly compared to spectral analysis based on the entire spectral range. Moreover, if certain spectral ranges have interferences or confounding factors (e.g., blood) etc., the effect of these can efficiently be reduced.

In an example a patient scheduled for endoscopic screening of suspicious symptoms for esophageal reflux undergoes the following:
- Conventional WLR/NBI/AFI endoscopic imaging is performed in the distal esophagus that shows inconclusive appearance of large Barrett's segments suspicious for pre-cancer (i.e., dysplasia).
- The broadband confocal Raman endoscope technique is subsequently applied to objectively target biopsies in the suspicious tissue segments.
- Confocal Raman classification is defined into "normal" (absence of pathology or gastritis), "low risk" (intestinal-metaplasia) and "high risk" (dysplasia/cancer).
- The confocal Raman probe is placed against the tissue in the distal esophagus and diagnosis is given in real-time based on complementary information extracted from the FP and HW region.
- The confocal Raman endoscope technique targets high-risk tissue sites that are subsequently biopsied.

Embodiments in accordance with the present disclosure demonstrate that real-time simultaneous fingerprint (FP) and high-wavenumber (HW) fiber-optic confocal Raman spectroscopy can be performed during screening of patients *in vivo.* Fiber-optic confocal Raman spectroscopy uncovers the bio-molecular and bio-chemical changes, such as protein, DNA, lipid and water occurring in the epithelium during colorectal carcinogenesis. The use of complementary bio-molecular information from the FP and HW range improves the detection of abnormal growths compared to using either the FP or HW ranges alone. The FP and HW fiber-optic confocal Raman spectroscopy technique has a great potential to improve pre-cancer and cancer detection and characterization. Use of a subset of the whole spectrum has a notable advantage in processing and in the identification of abnormal growths in many different parts of the body. The FP and HW ranges have been particularly identified as yielding good results for the types of detection discussed herein. It will be appreciated for other types of detection other ranges may prove more useful.

Embodiments in accordance with the present disclosure are not confined to biomedical Raman spectroscopy, but can also have application in other areas. These includes for example, fluorescence spectroscopy, elastic scattering spectroscopy, surface enhanced Raman spectroscopy, process analytical technology, water and environment monitoring, pharmaceutical process/drug delivery control, food industry, quality control industry, forensics, etc. In such embodiments, excitation energy provided by an illumination source is directed to a target sample (e.g., a chemical, water, or environmental material / substance sample, a pharmaceutical / drug or food sample, or another type of sample), which need not include or be tissue. Furthermore, such embodiments need not involve an endoscope or endoscopy. Reference to the term Raman herein is intended to include other types of spectroscopy including those mentioned above.

Embodiments in accordance with the present disclosure can serve as a diagnostic platform for any organs, such as the lung, upper and lower Gls (e.g. esophagus, stomach, colorectum), liver, bladder, head and neck (e.g., nasopharynx, larynx, oral cavity), cervix, skin, bone, or any other place where a conventional endoscope, laparoscope, or arthroscope can be used.

Some of the tissue Raman spectra obtained using certain embodiments in accordance with the present disclosure may suffer from endoscope illumination interferences. This can make diagnosis of abnormal growths difficult to achieve. As a result, a further embodiment offers a solution to eliminate such interferences.

A trimodal endoscope imaging system, according to an embodiment of the present disclosure, used to guide the confocal Raman fiber-probe as described above, includes a 300W short-arc xenon light source, a gastrointestinal (GI) endoscope, and a video signal processor. The xenon light source is coupled with different sets of filters to provide different illumination light(s) for trimodal endoscopic imaging in tandem (not shown). The filters can include, for instance: WLR (red filter, 585-655nm; green filter, 500-575nm; blue filter, 390-495nm), AFI (blue filter, 390-470nm; green filter, 540-560nm for reflectance image normalization), and NBI (green filter, 530-550nm; blue filter, 390-445nm).

The light reflected or fluorescence emitted from tissue is detected using two different CCDs mounted at the distal tip of the GI endoscope (not shown): one a conventional CCD for WLR/NBI and one a high-sensitivity CCD for AFI observation. The endoscope short-arc xenon lamp emits broadband continuous light covering UV/VIS/NIR with prominent discrete peaks in the NIR spectral range > 700nm. Since the 785nm laser excitation Stokes Raman spectroscopy also falls in the NIR range, ambient xenon light can interfere with the tissue Raman signal and fully obscure the *in vivo* tissue measurements and diagnosis. For this reason, Raman endoscopic diagnosis is conventionally performed with xenon illumination light turned 'off' or dimmed, which is highly undesirable in clinical settings.

To remove the ambient xenon illuminations interference, an embodiment is proposed in which the integration of a hot mirror low-pass filter (cutoff at ∼700 nm, ∼95% average transmission in visible range) in front of the xenon light source in the endoscope system enables elimination of ambient interference.

To demonstrate the application of this embodiment, *in vivo* Raman spectra are measured under different endoscopic illumination conditions. Figure 29A shows the non-contact tissue Raman spectrum obtained during endoscopy without the hot mirror integrated. Major interferences from the xenon light can be discerned fully overwhelming the tissue Raman signal. Figure 29B shows the tissue Raman spectrum in non-contact mode after integration of a hot mirror low-pass filter in front of the xenon lamp. As can be seen, xenon interferences are substantially eliminated. Highly resolved tissue Raman peaks can now be observed with tentative molecular assignments as follows:
- 853 cm⁻¹ which relates to v(C-C) proteins,
- 1004 cm⁻¹ which relates to vₛ(C-C) ring breathing of phenylalanine,
- 1078 cm⁻¹ which relates to v(C-C) of lipids,
- 1265 cm⁻¹ which relates to amide III v(C-N) and δ(N-H) of proteins,
- 1302 cm⁻¹ (which relates to CH₃CH₂ twisting and wagging of proteins,
- 1445 cm⁻¹ which relates to δ(CH₂) deformation of proteins and lipids,
- 1655 cm⁻¹ which relates to amide I v(C=O) of proteins, and
- 1745 cm⁻¹ which relates to v(C=O) of lipids.

The Raman spectra was measured with the probe in contact with the tissue. Figure 30A shows the tissue Raman spectrum measured in contact mode in the absence of the hot mirror. Figure 30B shows the tissue Raman spectrum in contact mode after integration of a hot mirror low-pass filter in front of the xenon lamp. Overall, these results show that integration of the hot mirror can fully eliminate ambient xenon interferences from the *in vivo* tissue Raman spectra both in non-contact and contact-mode of the confocal Raman probe. Moreover, it is evident from the figures that the xenon interference is most prominent in non-contact mode of the Raman probe with tissue since more light couples into the probe head. This embodiment is therefore of particular value in organs such as larynx/bronchus where non-contact mode is preferred due to stimulation of the cough reflex or in patients with advanced tumors where contact of the Raman probe with tissue can induce bleeding and risk for spread of cancer cells.

It should be noted that the filtering of illumination light for guidance using a hot mirror is not limited to the endoscopic application but is more general for filtering any light or imaging modality used to guide NIR tissue spectroscopy (i.e., surface-enhanced Raman spectroscopy (SERS), NIR fluorescence or NIR reflectance) for internal or external organs (e.g., skin, cervix, bladder, gastric, esophagus, nasopharynx, larynx, oral cavity, colon, rectum, lung, etc.).

The concept has therefore been generalized in Figure 31, which shows a system 2100. The system 2100 includes a probe 2102 which can be brought into contact with a tissue 2104. The system further includes a NIR laser 2106, a spectrograph 2108, a CCD 2110. An edge long pass filter 2112 is located between the probe and the spectrograph and a band pass filter 2114 is located between the probe and the laser. A PC 2116 and associated software is used to control the system. The tissue is also illuminated by an illumination source 2118 via a hot mirror low pass filter 2120. It will be appreciated that this system may vary in terms of the elements which make up the system and the orientation and position of such elements, depending on the precise application for which this embodiment is used.

It is known that instrument standardization has fallen far behind the pace of advances in clinical Raman spectroscopy. Because tissue Raman spectroscopy is based on inherently weak and highly resolved peaks, the technology is very sensitive to instrumental changes. It is therefore of imperative to develop and employ techniques for testing/calibrating and standardizing Raman endoscopy instrumentation prior to clinical measurements in human patients to ensure that the *in vivo* diagnosis is reliable and consistent among different Raman systems.

Figure 32 shows the basic principle of testing the Raman endoscope before application in patients comprising (i) startup of system, (ii) test of Raman endoscopy system (iii) application of Raman endoscope in patients. A new apparatus and technique or method is proposed for system testing and calibrating a fiber-optic Raman endoscope. This includes an opto-mechanical device and a set of program instruction routines implemented in the Raman software for automatic system testing and calibration in fiber-optic Raman diagnostic applications. The calibration device consists of spectral calibration radiation sources (e.g., mercury and Argon lamps, NIR spectra emitters, laser light, etc.), light intensity radiation source, laser power meter, tissue phantom, together with automated opto-mechanical parts (e.g., stepper motor) and controllers for standardization of the Raman endoscopy system. The calibration device is an integral part of the clinical Raman endoscopy platform but can also be used as stand-alone device for general testing and calibration of fiber-optic comprises an enclosed stepper-motor-driven filter wheel 2302 such as the FW102C, Thorlabs Inc. Newton, NJ, USA, which has different samples 2304 integrated therein. The flexible fiber-optic Raman probe 2306 can be mounted in the calibration device as schematically illustrated in Figure 33.

The filter wheel rotation has been synchronized with laser excitation and acquisition in the clinical Raman software. Spectra are acquired from each sample in the filter wheel and stored. The steps performed in the calibration/testing routines comprise measurement of the CCD characteristics (i.e., temperature, dark current), laser excitation power, and fused silica fiber background, a fluorescent material for system response calibration /testing, a material for wavelength calibration /testing and finally a tissue phantom. The signals measured can be used for recalibrating the parameters (e.g., intensity response, wavelength accuracy, background noise, etc.) of the Raman endoscope system.

The routines and standard samples that have been integrated in the filter wheel of the calibration device will now be described along with examples of the signals obtained. Figure 34 shows the procedure (2400) for testing the Raman endoscope prior to use in patients using the calibration device. Signal analysis (i.e., failure detection or calibration) may be performed after each measurement or after all measurements have been completed (as shown in this specific example).

In a first instance a detector signal of the CCD is measured and temperature is logged (2402). This may include measuring multiple spectra (i.e., at 0...1 sec exposure time) of the signal intensity in the absence of laser excitation. The system subsequently verifies that the dark current is less than a maximum value which was stored earlier in a factory configuration file. This embodiment further includes ensuring that the dark current coefficient of variation, over a series of spectra, is less than a maximum value which was also stored earlier in the configuration file.

The laser excitation power at the tip of the fiber-optic Raman probe is also measured (2402) using an integrated laser-power meter to ensure that this is within the range that is less than the American National Standards Institute (ANSI) maximum permissible skin exposure limit (which is set at 785nm laser beam). This embodiment includes ensuring that the laser power is less than a maximum value which was stored earlier in the configuration file.

The filter wheel contains an empty slot with dark environment so that a fused silica fiber probe background signal can be measured (2404). Multiple spectra of the fiber probe backgrounds are measured with laser excitation and different exposure times (e.g., 0.1...1.0 s). These spectra contain information about the condition of the fiber-optic probe. For instance, if the fiber is damaged or the probe tip is contaminated, this could be reflected by an increase in Raman or fluorescence intensity.

An example of background signals for the broad-band Raman endoscopy probe is shown in Figure 35 for two different fiber-optic probes. This embodiment further includes ensuring that the coefficient of variation, over a series of spectra, is less than a maximum value which was stored earlier in the configuration file. The measured fiber-optic probe background is also stored in the memory for further processing (i.e., subtraction) from *in vivo* tissue Raman spectra during real-time processing.

A fluorescent glass that exhibits a broad stable fluorescence spectrum (e.g., chromium doped borosilicate glass, green glass, kopp2412 etc.) is also measured and stored for testing and/or calibrating the response and collection efficiency of the Raman endoscopy system (2406). The standard fluorescence glass is factory calibrated beforehand using a National Institute of Standards and Technology (NIST) tungsten lamp according to the procedure detailed in Figure 36. The response of the system can be compared with a response which was stored earlier in the configuration file. This embodiment further includes ensuring that the coefficient of variation, over a series of spectra, is less than a maximum value which was stored earlier in the configuration file. The spectrum of the response standard sample can be used to recalibrate the system to the factory calibration. Figure 37 shows examples of calibration functions derived from a fluorescent standard glass (i.e., kopp2412) using 2 different fiber-optic probes.

A material with well-defined narrow Raman peaks is also measured (2408). An example is polystyrene shown in Figure 38. The wavelength standard is used to assess if there has been a drift in the wavelength axis due to optical misalignment. The spectrum is compared (e.g., using correlation coefficients, or peak identification etc.) with a spectrum which was stored earlier in the configuration file. This includes test of resolution (i.e., full width of half maximum (FWHM)) and verification of peak positions. The spectrum of the material with well-defined narrow Raman peaks can also be used to realign the system to the previous factory alignment stored in the configuration file. Recalibration comprises a polynomial mapping (e.g., 3...5 order) between predefined peaks from the factory calibrated wavelength axis and corresponding peaks from the misaligned spectrum. An example is shown in Figure 39, illustrating the polynomial mapping of peaks after recalibration.

The filter wheel also contains a layered tissue phantom. The tissue phantom consists of a material with diffuse properties and/or layered tissue phantoms that exhibit well-known Raman peaks which are measured (2410). This embodiment further includes ensuring that the coefficient of variation, over a series of spectra, is less than a maximum value which was stored earlier in the configuration file. An example is given in Figures 40A-B which show a comparison of Raman spectra acquired from the two-layer phantom sandwich of polystyrene and polyethylene using different Raman probes. The Raman spectra of the layered tissue phantoms are used to verify the depth selectivity of the fiber-optic Raman probe as well as spectrum quality. The ratio of the signal from the top to bottom layer (e.g., 1002 cm⁻¹ and 1296 cm⁻¹ respectively) is used as a qualitative and quantitative indicator of the depth selectivity for a given fiber-optic confocal probe. This embodiment includes ensuring that the depth selectivity is less than a maximum value which was stored earlier in the configuration file.

After the various measurements have been made, analysis of the signals is carried out (2412). This is to identify problems such as failure detection, calibration problems etc. If problems are detected the system is defined as not passing (2414). Alternatively system is passed if no problems are encountered (2416).

A further embodiment includes a program instruction set or software framework integrated with or as a GUI that is capable of displaying and saving Raman data on a spectrum and lesion basis in response to user selection(s) / input(s) when Raman endoscopic diagnosis is performed. For instance, if a first selection is made or a first button is pushed, Raman data from a first lesion will be saved. If a second selection is made or a second button is pushed, Raman data from a second lesion will be saved etc. Such a system also includes storing patient information, integration time, laser excitation power, time, date, diagnosis, probe background signal, system calibration function, endoscopy video and a logging-file containing measurement details. An integrated foot pedal device can be provided for interfacing and saving data by the clinician. If the footswitch is pushed, the data acquired will be stored as a first lesion. If pushed a second time, the data will be stored as a second lesion etc. The footswitch can also contain a safety pedal for turning on and off the laser excitation.

A further embodiment includes a program instruction set or software framework for displaying clinical Raman diagnosis together with the recorded wide-field video imaging. Endoscopic video imaging (i.e., WLR/NBI/AFI) and video recording has been integrated into the Raman endoscope software. Both the wide-field endoscopic video and the *in vivo* Raman diagnosis of the tissue imaged can be displayed on a display device such as a computer screen. The endoscopy video is synchronized in time with the measured tissue Raman spectra. Therefore, Raman spectral diagnosis can be displayed simultaneously with video playback. This enables the clinician to trace-back for each patient precise correlation between Raman endoscopic diagnosis and the suspicious tissue site sampled. This integrated imaging and Raman endoscopy software is a substantial improvement in the clinical system enabling review of historical clinical data.

The limited dynamic range of CCDs and the weak tissue Raman signals remain challenging in Raman endoscopic applications since *in vivo* tissues exhibit varying degree of auto fluorescence. For some tissues (e.g., gastric, lung, dorsal tongue, liver), detector saturation can occur in 0.1 second or even less than 0.05 second. For these tissues, the Raman signal can have prominent noise compared to other tissue types (e.g., esophagus, nasopharynx, larynx, cervix etc.). In general the Raman signal intensity of tissue scales linearly with laser excitation power and exposure time. The signal to noise ratio (S/N) is proportional to the square root of the exposure time. Currently, the user adjusts exposure time or laser excitation powers manually for every spectrum acquired using the Raman endoscopy technique. This can be highly impractical in real-time applications. Hence it is critical to define automated methods to prevent CCD saturation and assure that the Raman spectrum is acquired with optimum S/N ratio within times that are acceptable in clinical conditions. The realization of true real-time diagnosis is required for Raman endoscopy technology to gain widespread acceptance in clinical medicine. The novel method to automatically adjust laser excitation power, exposure time and spectrum accumulations to realize uninterrupted real-time diagnosis during clinical Raman measurements with high S/N ratio is invaluable in this quest. Two automatized techniques or methods are set out by way of representative example.

In a first technique or method as shown with reference to Figure 41, the exposure time is fixed (e.g., at 0.1 sec) (3102) and laser excitation power is assigned as variable and adjusted as required (3104). The Raman spectra are measured (3106) and signal to noise is determined (3108). Noise estimation in the spectra is based on Fourier transform, differentiation or other methods to quantify the noise level. If the Raman spectra have a poor signal to noise (S/N) ratio (3110), in the subsequent measurement, several Raman spectra (e.g., n=2...3) (3112) are accumulated before a read-out, If the CCD is saturated for a given spectrum (3114), laser excitation power of subsequent Raman measurement is reduced by a number according to the degree of saturation (3116). If the spectrum is not saturated it is used for diagnosis (3118). The degree of saturation is defined as a %age of saturated pixels. If the spectrum has been read out with intensity counts (<70% or >90% of dynamic range) (3120) the laser excitation power of the subsequent Raman measurement is scaled up so that the signal intensity will lie within 70-90% of the dynamic range (3122). One embodiment imposes an upper limit on the laser excitation power (e.g., 25 mW.). In another embodiment accumulation of several spectra (e.g., n=2) is consistently performed. Background subtraction, preprocessing, outlier detection, disease prediction and display may all be carried out (3124).

In a second technique or method as shown with respect to Figure 42, the laser excitation power is fixed (e.g., at 25 mW) (3202) and exposure time is assigned as a variable (3204). The Raman spectra are measured (3206) and signal to noise is determined (3208). Noise estimation is based on Fourier transform, differentiation or any method to quantify the noise level. If the Raman spectrum exhibits poor S/N ratio (3210), in the subsequent measurement, several Raman spectra (e.g., n=2...3) (3212) are accumulated before read-out. If the CCD is saturated (3214) for a given spectrum, exposure time of subsequent Raman measurement is reduced (3216) with a number according to degree of saturation. If the spectrum is not saturated it is used for diagnosis (3218). The degree of saturation is defined as the %age of saturated pixels. If spectrum has low number of intensity counts (e.g., <70% or >90% of dynamic range) (3220), the exposure time of the subsequent Raman measurement is scaled up (3222) so that the signal intensity will lie within 70-90% of the dynamic range. One embodiment imposes an upper limit on the exposure time (e.g., 0.5 sec.). In another embodiment accumulation of several spectra is consistently performed if the integration time is very low (e.g., <0.1s). Background subtraction, preprocessing, outlier detection, disease prediction and display may all be carried out (3224).

The above two functions detailed in respect of Figures 36 and 37 respectively are integrated in the operating software. Adjusting only the laser-power has the advantage that exposure time is kept constant and can be very low (e.g., 0.1 sec). The above methods are of significant value in clinical Raman endoscopy to realize uninterrupted real-time diagnosis with high S/N ratio, but also generalize to other modalities such as Surface Enhanced Raman Endoscopy (SERS), reflectance and fluorescence spectroscopy. The general conception of automated adjustment of exposure time, laser excitation power and accumulation to improve signal quality, prevent CCD saturation and realize real-time diagnosis is one important feature of this embodiment of the present disclosure. Another embodiment includes synchronization of the laser excitation with the acquisition. In other words, before signal acquisition, the laser excitation is turned on and after acquisition laser excitation is turned off.

A still further embodiment in accordance with the present disclosure includes the capability of switching between different fiber-optic probes and different organs. For each type of fiber-optic probe (e.g., confocal, or volume type probe) a database exists with diagnostic models specific to each organ (i.e., larynx, colon, nasopharynx, gastric, esophagus, oral cavity, skin, cervix, bladder etc.). For instance, if a user choses nasopharynx as an organ, one or more models belonging to the nasopharynx are loaded into the system. A representative database structure is shown in Figure 43. The model(s) can include multivariate diagnostic algorithms and/or multivariate outlier detection models.

It will be appreciated that the various embodiments described above are not intended to be limitative in their interpretation. Instead the invention is defined in the appended claims. Some of the processes may be implemented in hardware, software or any combination thereof.

### Listofreferencesigns

- 100: system - fiber-optic confocal Raman spectroscope
- 102: diode laser
- 104: imaging spectrograph
- 105: elongate shaft
- 106: camera
- 108: probe
- 110: optical fiber
- 112: band pass filter
- 114: long pass filter
- 116: tip
- 124: microcontroller system
- 200: apparatus
- 202: diode laser
- 204: reflective spectrograph
- 206: camera
- 208: fiber-optic confocal Raman probe
- DNA: deoxyribonucleic acids
- HW: high wave number regime
- FP: fingerprint
- NIR: near infrared
- GUI: graphical user interface
- PCA: principal components analysis
- SVM: support vector machine
- LR: logistic regression
- LDA: linear discriminant analysis
- PLS: partial least squares
- DA: discriminant analysis
- LVs: latent variables
- PEG: polyethylene glycol
- ACO: ant colony optimization
- CART: classification and regression trees
- AdaBoost: adaptive boosting
- H&E: hematoxylin and eosin
- ANOVA: analysis of variance
- LSD: least significant differences
- ROC: receiver operating characteristic
- AUCs: area under the curves
- SD: standard deviation
- IM: intestinal metaplasia
- CCD: charge-coupled device
- GI: gastrointestinal
- SERS: surface-enhanced Raman spectroscopy
- NIST: National Institute of Standards and Technology
- FWHM: full width of half maximum

## Claims

1. A Raman spectroscopy apparatus (2100) comprising:
a first illumination source (2106) configured for directing a collimated ilumination output into a tissue (2104);
a Raman spectrograph (2108) configured for simultaneously detecting fingerprint (FP) and
high wavenumber (HW) Raman spectra from illumination scattered by the tissue; and
a computerized control and analysis module (2116) comprising at least one processing unit and a memory storing program instructions executable by the at least one processing unit for analyzing discrete spectral sub-intervals of the Raman spectra in FP and HW wavenumber ranges to identify a match with specific peaks in the detected Raman spectra of one or both wavenumber anges,
**characterized by** further comprising:
an additional illumination source (2118) configured for outputting additional illumination into
the tissue while the first illumination source is directing collimated illumination into the tissue and
a hot mirror filter (2120) configured for compensating for illumination interference between the collimated illumination output by the first illumination source (2106) and the additional illumination output by the additional illumination source (2118).

2. The apparatus (2100) according to claim 1, **characterized in that** the Raman spectrograph (2108) has a single broadband diffraction grating, and wherein the apparatus (2100) further comprises a probe (2102) for transmitting the collimated illumination to the tissue and returning the Raman spectra from the tissue to the spectrograph (2108).

3. The apparatus (2100) according to claim 2, **characterized in that** the computerized control and analysis module (2116) includes program instructions executable by the at least one processing unit for diagnosing an abnormal growth based upon the match.

4. The apparatus (2100) according to claim 2, **characterized in that** the probe (2102) comprises a confocal fiber-optic probe and, optionally, an endoscope having an elongate shaft having an instrument channel within which the probe (2102) is carried.

5. The apparatus (2100) according to claim 2, **characterized in that** the computerized control and analysis module (2116) includes program instructions executable by the at least one processing unit for dynamically adjusting a power of the collimated illumination.

6. The apparatus (2100) according to claim 2, **characterized in that** the computerized control and analysis module (2116) includes program instructions executable by the at least one processing unit for dynamically adjusting a time to which the tissue is exposed to the collimated illumination.

7. The apparatus (2100) according to claim 2, **characterized by** further comprising a calibration apparatus configured for standardizing the probe (2102) or the entire Raman apparatus with respect to at least one calibration reference.

8. A Raman spectroscopy method performed by a Raman spectroscopy apparatus (2100), the method comprising:
directing collimated illumination output by a first illumination source (2106) into a tissue (2104);
simultaneously detecting by way of a Raman spectrograph (2108) fingerprint (FP) and high wavenumber (HW) Raman spectra from illumination scattered by the tissue;
analyzing discrete spectral sub-intervals in the detected Raman spectra in both FP and HW wavelength ranges; and
identifying a match with one or more reference markers in one or both wavenumber ranges;
**characterized by** further comprising:
directing additional illumination into the tissue using an additional illumination source (2118) while directing the collimated illumination output by first illumination source (2106) into the tissue; and
compensating for illumination interference between the collimated illumination output by the first illumination source (2106) and the additional illumination output by the additional illumination source (2118) using a hot mirror filter (2120).

9. The method according to claim 8, **characterized in that** simultaneously detecting FP and HW Raman spectra comprises diffracting illumination in both FP and HW wavelength ranges using a single broadband diffraction grating.

10. The method according to claim 8, **characterized by** further comprising dynamically adjusting the power of the illumination and/or adjusting a time to which the tissue is exposed to the illumination.

11. The method according to claim 8, **characterized by** further comprising performing a calibration or standardization procedure to standardize the Raman apparatus with respect to at least one calibration reference prior to illuminating the tissue.

## Patentansprüche

1. Raman-Spektroskopie-Vorrichtung (2100), umfassend:
Eine erste Lichtquelle (2106), die konfiguriert ist, eine kollimierte Lichtausgabe in ein Gewebe (2104) zu leiten;
einen Raman-Spektrographen (2108), der konfiguriert ist, gleichzeitig Fingerprint- (FP) und Hochwellenzahl- (HW) Raman-Spektren in dem Streulicht aus dem Gewebe zu erfassen; und
ein computergestütztes Steuer- und Analysemodul (2116) mit mindestens einer Verarbeitungseinheit und einem Speicher, in dem Programmbefehle gespeichert sind, die von der mindestens einen Verarbeitungseinheit ausführbar sind, um diskrete spektrale Teilintervalle der Raman-Spektren in FP- und HW-Wellenzahlbereichen zu analysieren, um eine Übereinstimmung mit spezifischen Peaks in den erfassten Raman-Spektren eines oder beider Wellenzahlbereiche zu identifizieren,
**gekennzeichnet dadurch, dass** sie des Weiteren umfasst:
eine zusätzliche Lichtquelle (2118), die konfiguriert ist, das Gewebe mit zusätzlichem Licht zu bestrahlen, während die erste Lichtquelle das Gewebe mit kollimiertem Licht bestrahlt; und
einen Warmlichtspiegel-Filter (2120), der konfiguriert ist, Lichtinterferenz zwischen dem von der ersten Lichtquelle (2106) abgestrahlten kollimierten Licht und dem von der zusätzlichen Lichtquelle (2118) abgestrahlten zusätzlichen Licht zu kompensieren.

2. Vorrichtung (2100) nach Anspruch 1, **dadurch gekennzeichnet, dass** der Raman-Spektrograph (2108) ein einziges Breitband-Beugungsgitter aufweist, und wobei die Vorrichtung (2100) des Weiteren eine Sonde (82102) zur Übertragung des kollimierten Lichts in das Gewebe und zur Zurückleitung der Raman-Spektren aus dem Gewebe an den Spektrographen (2108) umfasst.

3. Vorrichtung (2100) nach Anspruch 2, **dadurch gekennzeichnet, dass** das computergestützte Steuer- und Analysemodul (2116) Programmbefehle enthält, die von der mindestens einen Verarbeitungseinheit ausführbar sind, um ein abnormales Wachstum auf Grundlage der Übereinstimmung zu diagnostizieren.

4. Vorrichtung (2100) nach Anspruch 2, **dadurch gekennzeichnet, dass** die Sonde (2102) eine konfokale faseroptische Sonde und fakultativ ein Endoskop mit länglichem Schaft umfasst, der einen Instrumentenkanal aufweist, in dem die Sonde (2102) geführt ist.

5. Vorrichtung (2100) nach Anspruch 2, **dadurch gekennzeichnet, dass** das rechnergestützte Steuer- und Analysemodul (2116) Programmbefehle enthält, die von der mindestens einen Verarbeitungseinheit ausführbar sind zur dynamischen Einstellung der Lichtleistung des kollimierten Lichts.

6. Vorrichtung (2100) nach Anspruch 2, **dadurch gekennzeichnet, dass** das rechnergestützte Steuer- und Analysemodul (2116) Programmbefehle enthält, die von der mindestens einen Verarbeitungseinheit ausführbar sind zur dynamischen Einstellung einer Zeit, zu der das Gewebe mit dem kollimierten Licht bestrahlt wird.

7. Vorrichtung (2100) nach Anspruch 2, **dadurch gekennzeichnet, dass** sie des Weiteren eine Kalibrierungsvorrichtung umfasst, die konfiguriert ist, die Sonde (2102) oder die gesamte Raman-Vorrichtung in Bezug auf mindestens eine Kalibrierungsreferenz zu standardisieren.

8. Raman-Spektroskopieverfahren, das von einer Raman-Spektroskopie-Vorrichtung (2100) durchgeführt wird, wobei das Verfahren die folgenden Schritte umfasst:
Leiten des von einer ersten Lichtquelle (2106) abgestrahlten kollimierten Lichts in ein Gewebe (2104);
gleichzeitiges Erfassen von Fingerprint- (FP) und Hochwellenzahl- (HW) Raman-Spektren in dem Streulicht aus dem Gewebe mittels eines Raman-Spektrographen (2108);
Analysieren diskreter spektraler Teilintervalle in den erfassten Raman-Spektren sowohl im FPals auch im HW-Wellenlängenbereich; und
Identifizieren einer Übereinstimmung mit einem oder mehreren Referenzmarker in einem oder beiden Wellenzahlbereichen;
**dadurch gekennzeichnet, dass** das Verfahren des Weiteren folgende Schritte umfasst:
Leiten zusätzlichen Lichts in das Gewebe unter Verwendung einer zusätzlichen Lichtquelle (2118), während das von der ersten Lichtquelle (2106) abgestrahlte kollimierte Licht in das Gewebe geleitet wird; und
Kompensieren von Lichtinterferenz zwischen dem von der ersten Lichtquelle (2106) abgestrahlten kollimierten Licht und dem von der zusätzlichen Lichtquelle (2118) abgestrahlten zusätzlichen Licht unter Verwendung eines Warmlichtspiegel-Filters (2120).

9. Verfahren nach Anspruch 8, **dadurch gekennzeichnet, dass** das gleichzeitige Erfassen von FP- und HW-Raman-Spektren die Beugung von Licht sowohl im FP- als auch im HW-Wellenlängenbereich unter Verwendung eines einzigen Breitband-Beugungsgitters umfasst.

10. Verfahren nach Anspruch 8, **gekennzeichnet dadurch, dass** es des Weiteren den Schritt des dynamischen Einstellens der Lichtleistung und/oder einer Zeit, zu der das Gewebe mit dem Licht bestrahlt wird, umfasst.

11. Verfahren nach Anspruch 8, **gekennzeichnet dadurch, dass** es des Weiteren den Schritt des Durchführens eines Kalibrierungs- oder Standardisierungsverfahrens umfasst, um die Raman-Vorrichtung vor Bestrahlung des Gewebes in Bezug auf mindestens eine Kalibrierungsreferenz zu standardisieren.

## Revendications

1. Appareil de spectroscopie Raman (2100) comprenant :
une première source d'éclairage (2106) configurée pour diriger un éclairage collimaté émis à l'intérieur d'un tissu (2104) ;
un spectrographe Raman (2108) configuré pour détecter simultanément des spectres Raman d'empreinte (FP) et de nombre d'onde élevé (HW) à partir de l'éclairage diffusé par le tissu ; et
un module de commande et d'analyse informatisé (2116) comprenant au moins une unité de traitement et une mémoire stockant des instructions de programme pouvant être exécutées par l'au moins une unité de traitement pour analyser des sous-intervalles spectraux discrets des spectres Raman dans les plages FP et de nombre d'onde HW pour identifier une correspondance avec des crêtes spécifique dans les spectres Raman détectés d'une ou des deux plages de nombre d'onde,
**caractérisé en ce qu'**il comprend en outre :
une source d'éclairage additionnel (2118) configurée pour émettre un éclairage additionnel à l'intérieur du tissu pendant que la première source d'éclairage dirige un éclairage collimaté à l'intérieur du tissu ; et
un filtre de miroir chaud (2120) configuré pour compenser une interférence d'éclairage entre l'éclairage collimaté émis par la première source d'éclairage (2106) et l'éclairage additionnel émis par la source d'éclairage additionnel (2118).

2. Appareil (2100) selon la revendication 1, **caractérisé en ce que** le spectrographe Raman (2108) présente un seul réseau de diffraction à large bande, et dans lequel l'appareil (2100) comprend en outre une sonde (2102) destinée à transmettre l'éclairage collimaté au tissu et à renvoyer les spectres Raman du tissu au spectrographe (2108).

3. Appareil (2100) selon la revendication 2, **caractérisé en ce que** le module de commande et d'analyse informatisé (2116) inclut des instructions de programme pouvant être exécutées par l'au moins une unité de traitement pour diagnostiquer une croissance anormale sur la base de la correspondance.

4. Appareil (2100) selon la revendication 2, **caractérisé en ce que** la sonde (2102) comprend une sonde confocale à fibres optiques et, éventuellement, un endoscope présentant une tige allongée présentant un canal d'instrument dans lequel la sonde (2102) est transportée.

5. Appareil (2100) selon la revendication 2, **caractérisé en ce que** le module de commande et d'analyse informatisé (2116) inclut des instructions de programme pouvant être exécutées par l'au moins une unité de traitement pour le réglage dynamique d'une puissance de l'éclairage collimaté.

6. Appareil (2100) selon la revendication 2, **caractérisé en ce que** le module de commande et d'analyse informatisé (2116) inclut des instructions de programme pouvant être exécutées par l'au moins une unité de traitement pour le réglage dynamique d'un moment auquel le tissu est exposé à l'éclairage collimaté.

7. Appareil (2100) selon la revendication 2, **caractérisé en ce qu'**il comprend en outre un appareil d'étalonnage configuré pour normaliser la sonde (2102) ou tout l'appareil Raman par rapport à au moins une référence d'étalonnage.

8. Procédé de spectroscopie Raman effectué par un appareil de spectroscopie Raman (2100), le procédé comprenant :
le fait de diriger un éclairage collimaté émis par une première source d'éclairage (2106) à l'intérieur d'un tissu (2104) ;
la détection simultanée par le biais d'un spectrographe Raman (2108) de spectres Raman d'empreinte (FP) et de nombre d'onde élevé (HW) à partir de l'éclairage diffusé par le tissu ;
l'analyse de sous-intervalles spectraux discrets dans les spectres Raman détectés dans les deux plages de longueur d'onde FP et HW ; et
l'identification d'une correspondance avec un ou plusieurs marqueurs de référence dans une ou les deux plages de nombre d'onde ;
**caractérisé en ce qu'**il comprend en outre :
le fait de diriger un éclairage additionnel à l'intérieur du tissu au moyen d'une source d'éclairage additionnel (2118) tout en dirigeant l'éclairage collimaté émis par la première source d'éclairage (2106) à l'intérieur du tissu ; et
la compensation de l'interférence d'éclairage entre le l'éclairage collimaté émis par la première source d'éclairage (2106) et l'éclairage additionnel émis par la source d'éclairage additionnel (2118) au moyen d'un filtre de miroir chaud (2120).

9. Procédé selon la revendication 8, **caractérisé en ce que** la détection simultanée de spectres Raman FP et HW comprend la diffraction de l'éclairage dans les deux plages de longueur d'onde FP et HW au moyen d'un seul réseau de diffraction à large bande.

10. Procédé selon la revendication 8, **caractérisé en ce qu'**il comprend en outre le réglage dynamique de la puissance de l'éclairage et/ou le réglage d'un moment auquel le tissu est exposé à l'éclairage.

11. Procédé selon la revendication 8, **caractérisé en ce qu'**il comprend en outre la mise en oeuvre d'une procédure d'étalonnage ou de normalisation pour normaliser l'appareil Raman par rapport à au moins une référence d'étalonnage avant l'éclairage du tissu.
